(19) Europäisches Patentamt / European Patent Office / Office européen des brevets

(11) **EP 4 335 865 A1**

(12) **EUROPEAN PATENT APPLICATION**

(43) Date of publication:
**13.03.2024 Bulletin 2024/11**

(21) Application number: **22194458.0**

(22) Date of filing: **07.09.2022**

(51) International Patent Classification (IPC):
*C07K 14/025* [(2006.01)]   *G01N 24/00* [(2006.01)]
*G01N 33/50* [(2006.01)]   *G01N 33/569* [(2006.01)]
*G01N 33/574* [(2006.01)]   *G01N 33/80* [(2006.01)]
*G01R 33/44* [(2006.01)]   *G01R 33/46* [(2006.01)]

(52) Cooperative Patent Classification (CPC):
**G01N 33/56983; C07K 14/005; G01N 33/5091;
G01N 33/574; G01N 33/57469; G01N 33/66;
G01N 33/80;** C12N 2710/22022; G01N 2333/025;
G01N 2400/00; G01R 33/46

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Eberhard-Karls-Universität Tübingen
72074 Tübingen (DE)**

(72) Inventors:
• **RUSTMEIER, Nils
72076 Tübingen (DE)**

• **STEHLE, Thilo
72076 Tübingen (DE)**

(74) Representative: **Kuttenkeuler, David
SKM-IP PartGmbB
Oberanger 45
80331 München (DE)**

Remarks:
The complete document including Reference
Table(s) and the Sequence Listing(s) can be
downloaded from the EPO website

(54) **INTERACTION BETWEEN A GLYCOLIPID AND MAJOR CAPSID PROTEIN (VP1) OF SHEEP POLYOMAVIRUS AND, METHODS, KITS AND USES THEREOF**

(57)    The invention is based on the interaction of glycolipid known as the Forssman antigen and the major capsid protein 1 of sheep polyomavirus (ShPyV-VP1). The invention surprisingly shows that the interaction of the glycolipid of the invention and ShPyV-VPi can be exploited to detect the presence of the glycolipid in a biological sample and thereby to diagnose certain mammalian blood types and/or physiological conditions such as diseases. The invention provides methods, uses and kits useful for an application in research, diagnostic and therapy.

FIGURE 3

EP 4 335 865 A1

**Description**

FIELD OF THE INVENTION

The invention is based on the interaction of glycolipid known as the Forssman antigen and the major capsid protein 1 of sheep polyomavirus (ShPyV-VP1). The invention surprisingly shows that the interaction of the glycolipid of the invention and ShPyV-VP1 can be exploited to detect the presence of the glycolipid in a biological sample and thereby to diagnose certain mammalian blood types and/or physiological conditions such as diseases. The invention provides methods, uses and kits useful for an application in research, diagnostic and therapy.

DESCRIPTION

Polyomaviruses (PyVs) are non-enveloped viruses that contain double-stranded DNA genomes of about 5 kilobase pairs. PyV genomes usually comprise up to nine genes for regulatory and structural proteins. During the early steps of viral replication, the large and small tumor antigens (LTag and sTag) regulate the viral genome replication. They are responsible for cellular transformation and hence the oncogenic potential of polyomavirus species such as simian virus 40, murine polyomavirus (mPyV), and Merkel Cell polyomavirus (MCPyV). 1-3 The capsid of polyomaviruses comprises minor and major capsid proteins expressed towards the end of the replicative cycle. 4-6 The major capsid protein VP1 forms pentameric capsomers and 72 of these capsomers assemble into the shell of the T=7d icosahedral capsid.

Most polyomaviruses investigated to date engage their target cells through interactions of the VP1 protein with sialylated glycoproteins or sialic acid-bearing gangliosides. 7-11 The sialic acid binding site of VP1 is located at the apical site of the pentamer and is exclusively formed by elaborate surface loops. Crystallographic studies have revealed six distinct orientations of sialic acids with varying intermolecular interactions to the VP1 surface 12-19. Thereby, specificity for a certain sialoside receptor is encoded by strategically placed additional contacts that recognize the structural context of the sialic acid, such as chemical modification, its linkage to subsequent sugar residues or the shape of the entire glycan. 20,21 Individual interactions between VP1 and sialoside receptors are weak and based on a small number of contacts, most of which are mediated by the terminal sialic acid itself. To secure cell attachment this low affinity is compensated by the avidity of 360 distinct binding sites on the virion surface. 22-24 Apart from sialylated cell membrane components, other host factors associated to polyomavirus infection comprise the serotonin receptor 5-HT$_2$ for JC polyomavirus (JCPyV) and glycosaminoglycans (GAGs) for Merkel Cell polyomavirus (MCPyV), BK polyomavirus (BKPyV), and JCPyV strains associated with Progressive Multifocal Leukoencephalopathy. 25-30 However, detailed structural information about the engagement of these molecules by polyomavirus capsid proteins is not yet available.

Recently, the inventors investigated the engagement of sialylated glycans by several human and animal polyomavirus VP1. The inventors showed that sheep polyomavirus (ShPyV), a betapolyomavirus isolated from commercial lamb meat, uses VP1 to recognize sialic acids in a binding site similar to that of the human pathogenic *Trichodysplasia* spinulosa-associated polyomavirus (TSPyV). 19,31-33 Here, the inventors have subjected ShPyV VP1 to extensive glycan microarray screening and identified the Forssman glycolipid as a non-ionic receptor candidate.

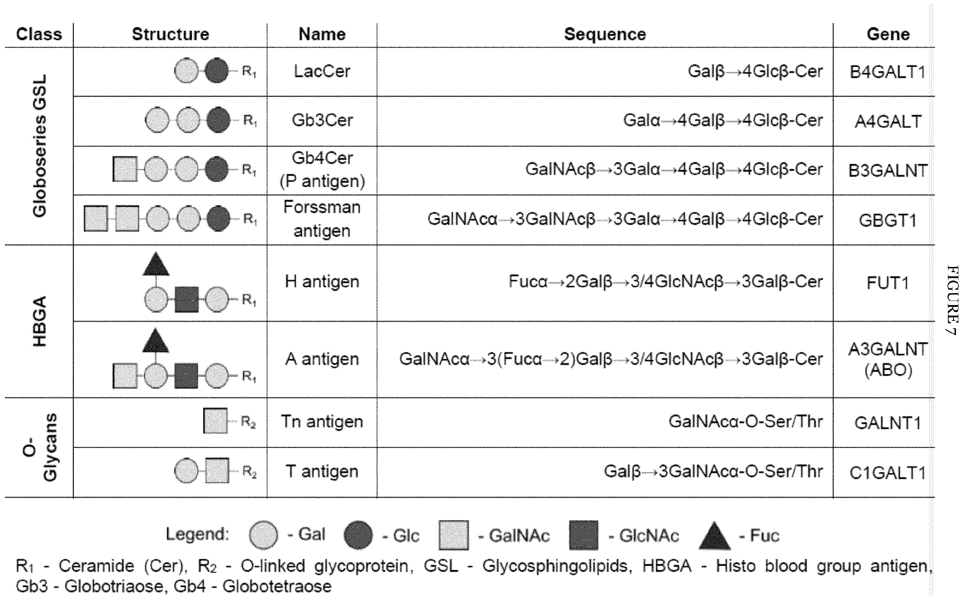

The heterophile Forssman antigen (FA) is a member of the neutral globoseries glycosphingolipids (GSL) succeeding globotriaosylceramide (Gb3Cer) and the P antigen globoside (Gb4Cer). During FA synthesis, the attachment of $\alpha$-*N*-acetylgalactosamine ($\alpha$-GalNAc) to Gb$_4$Cer is catalyzed by Forssman synthetase (FS) and results in the terminal GalNAc$\alpha\rightarrow$3GalNAc$\beta$ Forssman disaccharide motif. 34,35 The FS reaction is paralogous to the ABO glycosyltransferase A3GALNT activity. Thus, FA structurally resembles the human A antigen, which also carries a terminal $\alpha\rightarrow$3-linked GalNAc (Figure 7). 36,37 Despite the abundance of precursor GSL in humans, Forssman antigen is usually absent in human cell membranes due to an evolutionary inactivated FS. 38 FA is also absent in primates, rats, rabbits, cows, and pigs but present in various other mammals such as guinea pigs, horses, mice, goats, cats, dogs, and sheep. 38,39 Although the genetic background of Forssman expression is mostly resolved, physiological implications of its presence remain fragmentary. It was shown that Forssman antigen serves as a receptor for certain pathogens such as the *E. coli* virulence factors adhesin PapGIII in canines and mice and Shiga toxin (Stx) variant 2e in swine. 40-44 Elsewhere, FS activity correlates with a reduced cellular Stx susceptibility due to fewer precursor GSL, i.e. Gb4Cer, in the membrane. 45 Humans produce naturally occurring anti-Forssman antibodies with the exception of the few existing individuals of the rare FORS1+ blood group. 46,47 Reactivation and expression of FA was reported in certain human cancer types such as gastrointestinal, breast, and lung carcinomas. 48-57 Titers of anti-Forssman antibodies may decline in patients suffering from these cancers and again increase after successful surgical removal of the tumor. 52 In absence of recurrence, anti-Forssman titers eventually reached the level of the healthy control group. However, prior to recurrence, postsurgical antibody titers persistently remained below the native level. After recurrence, titers even dropped below presurgical levels and were accompanied by severe prognosis. 58

Therefore, an object of the invention to provide means for the detection of certain glycolipids, such as the Forssman antigen.

BRIEF DESCRIPTION OF THE INVENTION

Generally, and by way of brief description, the main aspects of the present invention can be described as follows:
In **a first aspect,** the invention pertains to a method for the detection of a target oligosaccharide antigen in a biological sample, the method comprising the steps of:

- Providing the biological sample suspected to contain the target oligosaccharide antigen and an antigen detection component, wherein the antigen detection component comprises:

  i) a major capsid protein (VP1) of sheep polyomavirus (ShPyV), or an antigen binding fragment thereof, or

  ii) a derivative of ShPyV-VP1 having an amino acid sequence which is at least 80% identical to the amino acid sequence of ShPyV-VP1 (SEQ ID NO:1), or an antigen binding fragment of the derivative of ShPyV-VP1 (such as SEQ ID NO 2 or 3);

- Bringing into contact the biological sample and the antigen detection component under conditions allowing for a specific binding between the target oligosaccharide antigen and the antigen detection component;

- Optionally; removal of unbound sample material,

- Detection of a binding between the target oligosaccharide antigen to the antigen detection component;

  wherein a detected specific binding in (d) indicates the presence of the target oligosaccharide antigen in the sample;

  characterized in that the target oligosaccharide antigen comprises at least a disaccharide GalNAc$\alpha\rightarrow$3GalNAc$\beta$.

In **a second aspect,** the invention pertains to an *in-vitro* method for the detection of an expression of the Forssman antigen in a human subject, the method comprising providing a biological sample of the human subject, and performing with the biological sample of the human subject the method for detection of a target oligosaccharide antigen in a biological sample according to the first aspect, and wherein the target oligosaccharide antigen is the Forssman antigen.
In **a third aspect,** the invention pertains to an *in-vitro* method of diagnosing a pathological condition in a human subject, the method comprising the steps of: providing a biological sample of the human subject, and performing with the biological sample of the human subject the method for detection of a target oligosaccharide antigen in a biological sample according to the first aspect, and wherein the target oligosaccharide antigen is the Forssman antigen, wherein the presence of the Forssman antigen in the biological sample indicates a pathological condition in the human subject.
In **a fourth aspect,** the invention pertains a kit for detecting a target oligosaccharide antigen, the kit comprising an antigen detection component comprising: i) a major capsid protein (VP1) of sheep polyomavirus (ShPyV), or an antigen binding fragment thereof, or (ii) a derivative of ShPyV-VP1 having an amino acid sequence which is at least 80% identical to the amino acid sequence of ShPyV-VP1 (SEQ ID NO:1), or an antigen binding fragment of the derivative of ShPyV-VP1 (such as preferably SEQ ID NO 2 or 3).
In **a fifth aspect,** the invention pertains to an antigen detection component comprising: i) a major capsid protein (VP1) of sheep polyomavirus (ShPyV), or an antigen binding fragment thereof, or (ii) a derivative of ShPyV-VP1 having an amino acid sequence which is at least 80% identical to the amino acid sequence of ShPyV-VT1 (SEQ ID NO:1), or an antigen binding fragment of the derivative of ShPyV-VP1 (such as preferably SEQ ID NO 2 or 3); wherein the antigen detection component comprises, or is coupled to, a detectable moiety.

DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to a method for the detection of a target oligosaccharide antigen in a biological sample, the method comprising the steps of:

- Providing the biological sample suspected to contain the target oligosaccharide antigen and an antigen detection component, wherein the antigen detection component comprises:

   i) a major capsid protein (VP1) of sheep polyomavirus (ShPyV), or an antigen binding fragment thereof, or

   ii) a derivative of ShPyV-VP1 having an amino acid sequence which is at least 80% identical to the amino acid sequence of ShPyV-VP1 (SEQ ID NO:1), or an antigen binding fragment of the derivative of ShPyV-VP1 (such as preferably SEQ ID NO 2 or 3);

- Bringing into contact the biological sample and the antigen detection component under conditions allowing for a specific binding between the target oligosaccharide antigen and the antigen detection component;

- Optionally; removal of unbound sample material,

- Detection of a binding between the target oligosaccharide antigen to the antigen detection component;

   wherein a detected specific binding in (d) indicates the presence of the target oligosaccharide antigen in the sample;

   characterized in that the target oligosaccharide antigen comprises at least a disaccharide GalNAc$\alpha$1$\rightarrow$3GalNAc$\beta$.

The term "oligosaccharide antigen" in accordance with the invention shall be understood as an antigenic compound comprising at least two monosaccharides that are covalently linked to each other, preferably in direct sequence. Preferred oligosaccharides are glycoproteins and/or glycolipids.

Glycoproteins include any proteins that contain covalently attached oligosaccharide chains (glycans). Glycans are attached to glycoproteins in a co-translational or posttranslational modification, known as glycosylation. Glycoproteins are present in the extracellular space as secreted molecules, cell surface as integral membrane proteins, or inside the cell. During glycosylation, carbohydrates are often linked to polypeptides through N-linked protein glycosylation (N-glycosylation of N-Glycans) on the amide nitrogen on the side-chain of asparagine (Asn) residues; or through O-linked protein glycosylation (0-glycosylation of O-Glycans) on the hydroxyl oxygen on the side-chain of hydroxylysine, hydroxyproline, serine or threonine residues. The sequences and sizes of oligosaccharide chains on glycoproteins are diverse.

As used herein, the term "glycolipid" refers to compounds composed of lipid that are covalently bound to one or more carbohydrate residues or glycans. Attached carbohydrate residues may include galactose, glucose, inositol, or others. Carbohydrate residues and glycans are usually bound to lipids by a glycosidic linkage to a hydrophobic moiety such as an acylglycerol, a sphingoid, a ceramide or a prenyl phosphate. Glycolipids can be categorized into several subtypes including glycoglycerolipids, which are glycolipids containing one or more glycerol residues; glycosphingolipids (GSLs) which are lipids containing at least one monosaccharide residue and either a sphingoid or a ceramide; glycophosphatidylinositol which are glycolipids which contain carbohydrate residues or glycans glycosidically linked to the inositol moiety of phosphatidylinositols (e.g. diacyl-sn-glycero-3-phosphoinositol), inclusive of lyso-species and those with various O-acyl-, O-alkyl-, O-alk-1-en-1-yl- (e.g. plasmanylinositols) or other substitutions on their glycerol or inositol residues; fucoglycosphingolipid; mannoglycosphingolipid; and xyloglycosphingolipid.

Glycolipids are primarily found in cell membranes as membrane components. Glycolipid-enriched membrane domains can be involved in different biological functions such as cell-cell adhesion, receptor mediated signal transduction and as targets for host pathogens and their toxin bindings.

As used herein, the term "proteoglycan" or "PG" refers to proteins that are heavily glycosylated in which the core protein/polypeptide is covalently bound to one or more glycosaminoglycan (GAG) chains. The GAG chains are attached through a tetrasaccharide bridge to serine residues of the core protein. Proteoglycans can be categorized depending on the nature of their glycosaminoglycan chains as chondroitin sulfate (CS)/dermatan sulfate (DS) proteoglycans (CS/DS-PGs) (e.g., decorin, biglycan, versican), heparan sulfate (HS)/chondroitin sulfate (CS) proteoglycans (e.g., testican, perlecan), chondroitin sulfate (CS) (e.g. bikunin, neurocan, aggrecan), herapan sulfate (HS) (e.g. syndecan, glypican) and keratan sulfate (e.g. fibromodulin, lumican). Proteoglycans are major components of the extracellular matrix forming large complexes.

In a preferred embodiment of the invention the oligosaccharide antigen is any compound comprising at least the disaccharide GalNAc$\alpha$1$\rightarrow$3GalNAc$\beta$, more preferably the Forssman pentose. In preferred embodiments of the invention the oligosaccharide antigen is or comprises the Forssman antigen. The Forssman antigen comprises a pentose coupled to a ceramide with the following structure GalNAc$\alpha$1$\rightarrow$3GalNAc$\beta$1$\rightarrow$3Gal$\alpha$1$\rightarrow$4Gal$\beta$1$\rightarrow$4Glc$\beta$1->1Cer.

The term "ShPyV-VP1" shall refer to a protein with the amino acid sequence shown in SEQ ID NO:1.

In a preferred embodiment antigen binding fragment of ShPyV-VP1, or the derivative of ShPyV-VP1, or antigen binding fragment thereof, when the amino acid sequence is aligned to ShPyV-VP1 (SEQ ID NO:1) comprises at least one, preferably at least two, three or all, of the amino acids in ShPyV-VP1 (SEQ ID NO:1): F53, F56, F256, (F66), Q266, D64, N268, Q47, N59. It is understood that amino acid positions indicated in brackets constitute an essential amino acid for the binding of the Forssman antigen in only one of the VP1 dimers necessary for providing the antigen binding site.

dMore preferably the antigen binding fragment of ShPyV-VP1, or the derivative of ShPyV-VP1, or antigen binding fragment thereof, when the amino acid sequence is aligned to ShPyV-VP1 (SEQ ID NO: 1) comprises at least one, preferably two or three, more preferably all of the amino acids in ShPyV-VP1 (SEQ ID NO: 1): D58, N59, and K270. In some embodiments, a derivative of ShPyV-VP1 is a protein having an amino acid sequence that does not comprise a sequence that is 100% identical to SEQ ID NO: 1. Preferably, such a derivative comprises an amino acid sequence that between 70%, more preferably 80%, and 99% identical, but not 100% identical, to the amino acid sequence of SEQ ID NO: 1. More preferably a derivative comprises an amino acid sequence that is at least 80%, more preferably 85%, 90%, 95% or more identical to any one of SEQ ID NO: 2 or 3. In certain embodiments, any of the herein described derivatives or variants of ShPyV-VP1 maintains an capability to assemble under physiological conditions as a pentameric protein complex composed of 5 monomers.

In another preferred embodiment, the ShPyV-VP1, or the derivative of ShPyV-VP1, or antigen binding fragment thereof, of the invention, is provided as a pentamer, either a homopentamer, or as a pentamer with non-identical ShPyV-VP1 monomers, or the derivative of ShPyV-VP1, or antigen binding fragment thereof of the invention.

Methods for detecting the binding between the target oligosaccharide antigen to the antigen detection component are well known to the skilled artisan and shall not be restricted in context of the present invention. However, in most preferred embodiments, the interaction is detected by saturation-transfer difference (STD) NMR, or by using a detectable moiety.

The term "detectable moiety" shall pertain to any attachable molecule that allows for a specific and selective detection of its presence. Such detection may involve that the antigen detection component comprises the detectable moiety, and or the use of secondary detection molecule comprising the detectable moiety and which specifically binds the major capsid protein (VP1) of sheep polyomavirus (ShPyV), or the antigen binding fragment thereof, or specifically binds the derivative of ShPyV-VP1), or the antigen binding fragment of the derivative of ShPyV-VP1. Preferably, the detectable moiety is selected from the group consisting of a fluorophore, a chromophore, a radionuclide, a chemiluminescent agent, a bioluminescent agent and an enzyme.

It is well known to the person of skill in the relevant art, that the antigen detection component is soluble or is bound to a solid matrix, such as an array or bead.

In **a second aspect,** the invention pertains to an *in-vitro* method for the detection of an expression of the Forssman antigen in a human subject, the method comprising providing a biological sample of the human subject, and performing with the biological sample of the human subject the method for detection of a target oligosaccharide antigen in a biological sample according to the first aspect, and wherein the target oligosaccharide antigen is the Forssman antigen.

A subject in context of the herein disclosed invention may be selected from a healthy subject, and the method is in these instances for example a method for determining the FORS1+ blood type in the (healthy) human subject. Of course healthy subject may suffer from other FORS+ unrelated conditions that are not subject of the present invention.

The term "biological sample" may pertain to a liquid or solid sample obtained from a subject, such as a mammal, preferably a human. Liquid samples in accordance with the invention are blood, or blood derived samples, such as plasma or serum samples. However, the invention may also be practised using other samples derived from biological fluids which is well known in the pertinent art.

In **a third aspect,** the invention pertains to an *in-vitro* method of diagnosing a pathological condition in a human subject, the method comprising the steps of: providing a biological sample of the human subject, and performing with the biological sample of the human subject the method for detection of a target oligosaccharide antigen in a biological sample according to the first aspect, and wherein the target oligosaccharide antigen is the Forssman antigen, wherein the presence of the Forssman antigen in the biological sample indicates a pathological condition in the human subject.

A pathological condition in context of the present invention may be characterized by an abnormal expression and/or presence of the Forssman antigen in the human subject.

Preferably the pathological condition is a susceptibility of the subject to an infection with a pathologic microorganism.

In some embodiments of the invention the pathological condition is cancer, such as a cancer disease characterized by the expression of the Forssman antigen in cells associated with the cancer disease, for example wherein the cancer disease is Forssman positive gastrointestinal cancer, a lung cancer or a breast cancer.

In **a fourth aspect,** the invention pertains a kit for detecting a target oligosaccharide antigen, the kit comprising an antigen detection component comprising: i) a major capsid protein (VP1) of sheep polyomavirus (ShPyV), or an antigen binding fragment thereof, or (ii) a derivative of ShPyV-VP1 having an amino acid sequence which is at least 80% identical to the amino acid sequence of ShPyV-VP1 (SEQ ID NO:1), or an antigen binding fragment of the derivative of ShPyV-VP1.

In **a fifth aspect,** the invention pertains to an antigen detection component, and its uses, the antigen detection component comprising: i) a major capsid protein (VP1) of sheep polyomavirus (ShPyV), or an antigen binding fragment thereof, or (ii) a derivative of ShPyV-VP1 having an amino acid sequence which is at least 80% identical to the amino acid sequence of ShPyV-VP1 (SEQ ID NO:1), or an antigen binding fragment of the derivative of ShPyV-VP1; wherein the antigen detection component comprises, or is coupled to, a detectable moiety.

Preferably, in some embodiments of the invention, antigen binding fragment of ShPyV-VP1, or the derivative of ShPyV-VP1, or antigen binding fragment thereof, when the amino acid sequence is aligned to ShPyV-VP1 (SEQ ID NO: 1) comprises at least one, preferably at least two, three or all, of the amino acids in ShPyV-VP1 (SEQ ID NO: 1): F53, F56, F256, (F66), Q266, D64, N268, Q47, N59. It is understood that amino acid positions indicated in brackets constitute an essential amino acid for the binding of the Forssman antigen in only one of the VP1 dimers necessary for providing the antigen binding site.

Preferably, an antigen binding fragment of ShPyV-VP1, or the derivative of ShPyV-VP1, or antigen binding fragment thereof, when the amino acid sequence is aligned to ShPyV-VP1 (SEQ ID NO: 1) comprises at least one, preferably two or three, more preferably all of the amino acids in ShPyV-VP1 (SEQ ID NO: 1): D58, N59, and K270.

In some embodiments of the invention, the antigen detection component comprises, or is coupled to, a detectable moiety, as described herein before. Preferably, the detectable moiety is selected from the group consisting of a fluorophore, a chromophore, a radionuclide, a chemiluminescent agent, a bioluminescent agent and an enzyme.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by $\pm 20\%$, $\pm 15\%$, $\pm 10\%$, and for example $\pm 5\%$. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

BRIEF DESCRIPTION OF THE FIGURES AND SEQUENCES

The figures show:

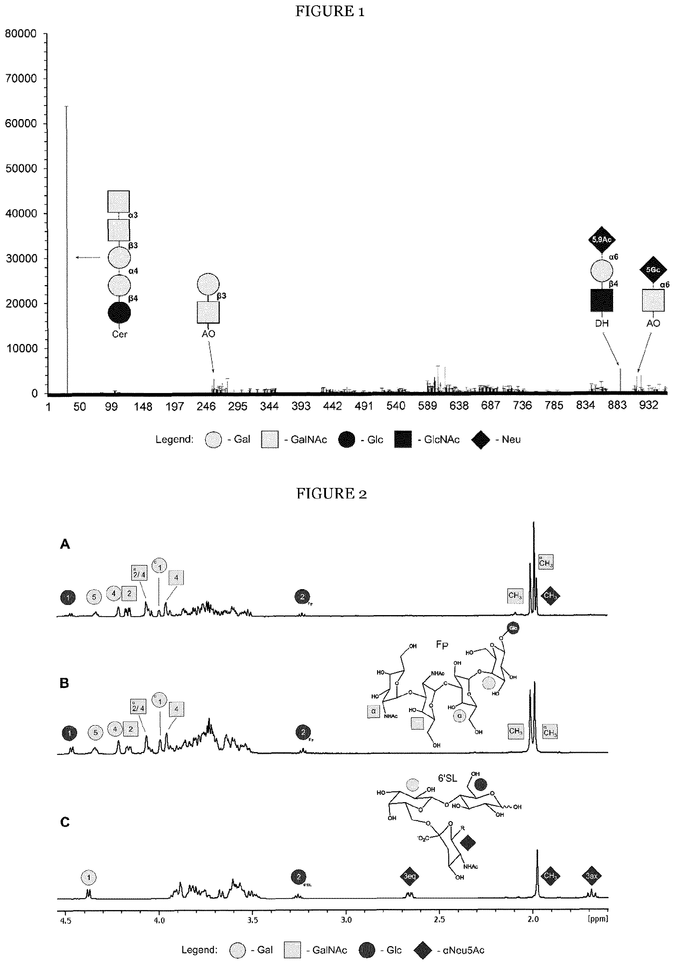

**Figure 1: Glycan mirco-array screening of VP1 reveals high-fidelity engagement of Forssman glycolipid.** Binding intensities are displayed with error bars corresponding to half of the respective value difference.

**Figure 2: Sheep polyomavirus VP1 interacts with Forssman pentaose in solution.** The STD-NMR spectrum of VP1 in presence of Forssman pentaose ($F_P$) and 6'sialyllactose (6'SL) shows responses for the binding of both glycans to the protein **(A)**. $^1$H references spectra of isolated $F_P$ **(B)** and 6'SL **(C)** are provided. All $^1$H resonances are labeled according to their carbon position in the monosaccharides and the respective monosaccharide symbol.

Signals stemming from $\alpha$-linked monosaccharide units in $F_P$ are labeled as such. Spectra are displayed in the high field from 4.5-1.6 parts per million (ppm).

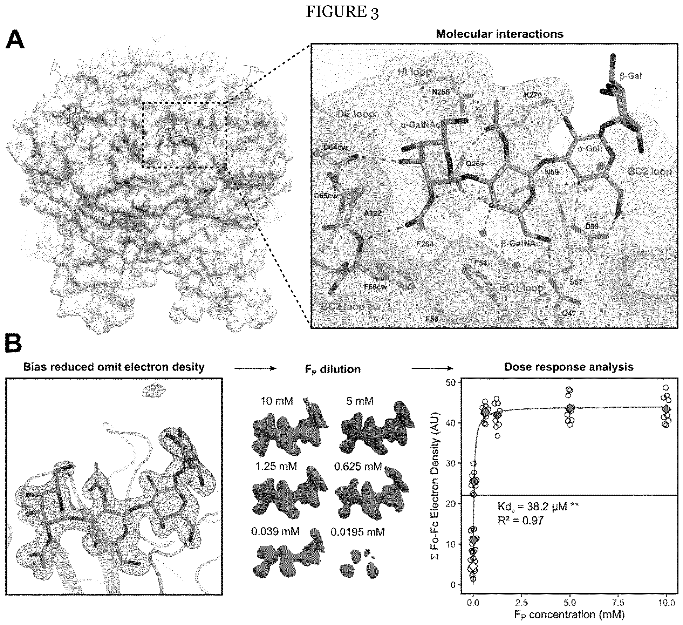

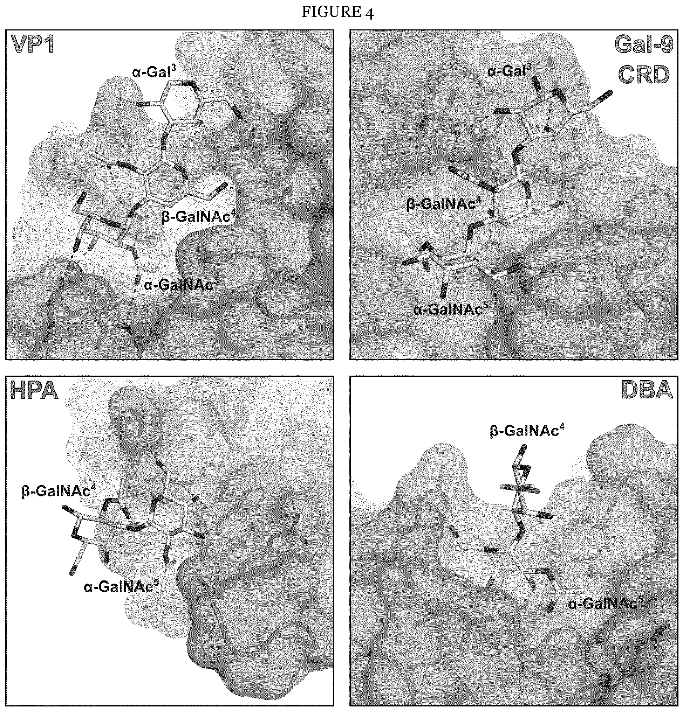

**Figure 3: Forssman pentaose binds to the outer surface of ShPyV VP1 with multiple contacts governing a millimolar crystallographic dissociation constant. (A)** Five Forssman pentaose ($F_P$) binding sites are exposed on top of a single VP1 pentamer (left) and the intermolecular contacts to $F_P$ involve the terminal trisaccharide sequence of the oligosaccharide (right). **(B)** The bias-reduced Fo-Fc omit electron density is displayed at a level of 2.5 $\sigma$ and with a radius of 1.6 Å around a single glycan molecule in the 10 mM FP data set (left). Omit electron densities of diluted $F_P$ concentrations in ShPyV VP1 crystals are displayed similarly (middle). Analysis of the dose response data using a Michaelis-Menten approximation (Methods) was used to determine the crystallographic dissociation constant ($Kd_c$) **(left).Figure 4:** Structure model of ShPyV VP1 derivatized with FA glycan. A) The single VP1 pentamer in cartoon representation with one chain highlighted in light orange with liganded FA glycan drawn as sticks. B) Close up view of a singular binding site with an additional simulated annealing omit Fo-Fc electron density map (blue mesh), displayed at a contour level of 2.5 $\sigma$ and 1.6 Å around the oligosaccharide chain. PDB ID 7B6S.

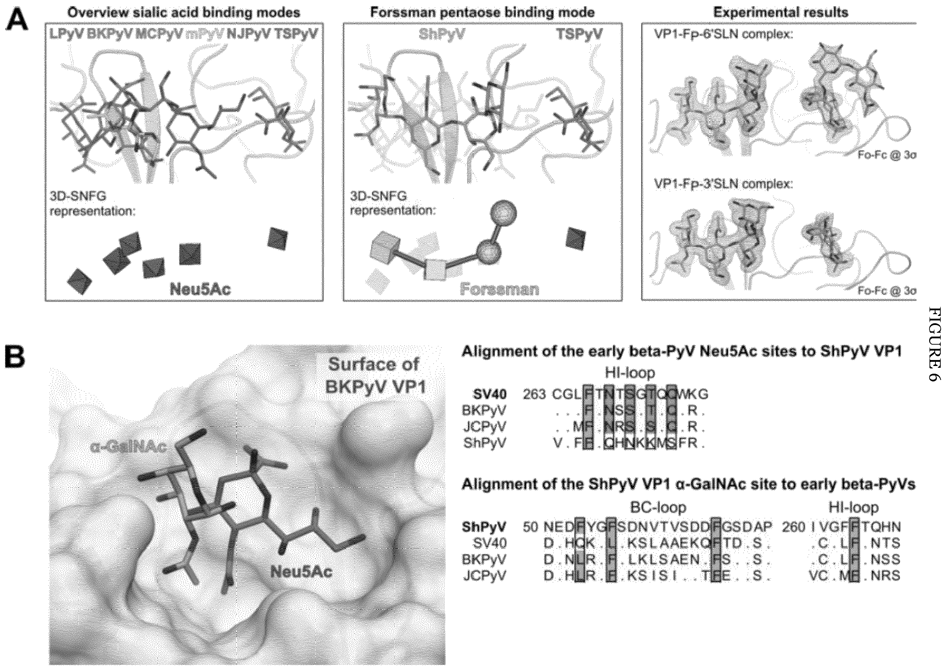

**Figure 4: Comparison of the Forssman glycan recognition modes for in different lectins.** VP1 recognizes a trisaccharide sequence of Forssman pentaose ($F_P$) with several hydrogen bonds to each unit (upper left panel). Human Galectin-9 carbohydrate recognition domain (Gal-9 CRD, PDB 5X4A) mainly interacts with $\beta$-GalNAc[4] and $\alpha$-Gal[3] of $F_P$ (upper right panel). *Helix pomatia* agglutinin (HPA, PDB 2CGY) and *Dolichos biflorus* agglutinin (DBA, PDB 1LU1) bind the Forssman disaccharide epitope via hydrogen bonds to the terminal $\alpha$-GalNAc[5] but not the subsequent $\beta$-GalNAc[4]. **Figure 6:** Michaelis-Menten analysis of bias-reduced simulated annealing omit Fo-Fc electron densities of Forssman pentaose $F_P$. Density integrals of the ten individual chains of VP1 are depicted as dots and plotted against the $F_P$ concentration.

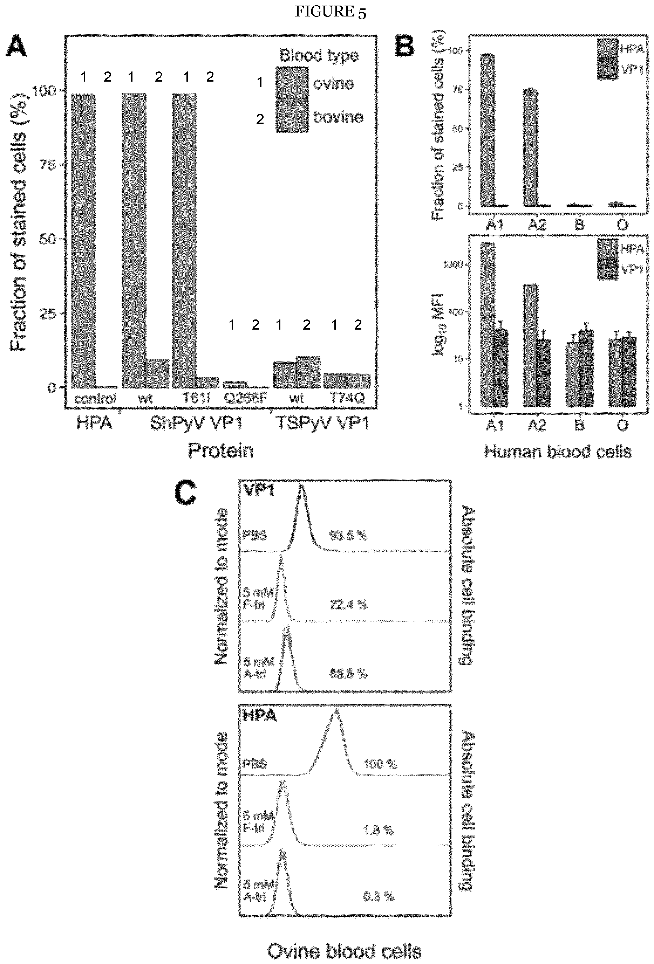

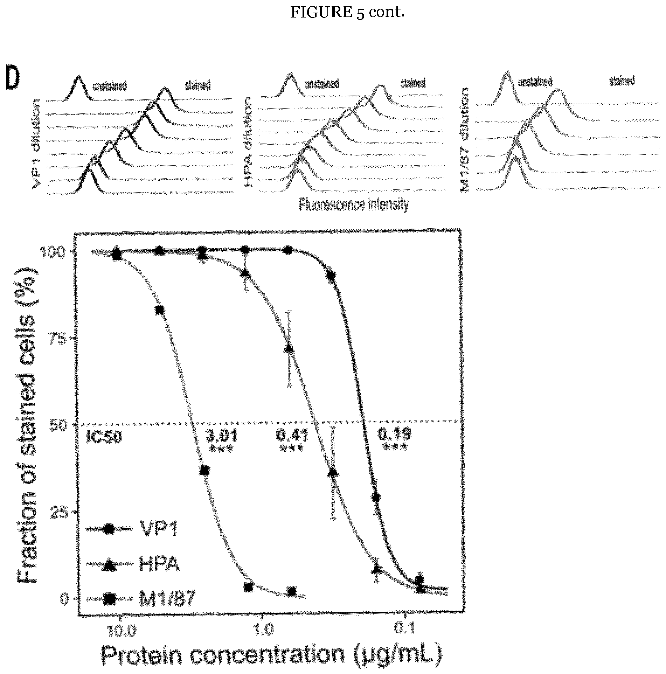

**Figure 5: Flow cytometry analyses of Forssman lectin binding to erythrocytes.** A) Absolute binding of wild-type (wt) and mutated VP1 of ShPyV and human Trichodysplasia spinulosa-associated polyomavirus (TSPyV) to sheep (ovine) and cattle (bovine) blood cells. Helix pomatia agglutinin (HPA) serves as a control for aGalNac-epitope recognition. B) Comparison of wt ShPyV VP1 and HPA binding to human ABO type erythrocytes with stained fractions (above) and median fluorescence intensity (below). C) Inhibition assay of ShPyV VP1 and HPA binding to ovine blood cells. PBS serves as negative control and the trisaccharides of Forssman (F-tri) and A antigen (A-tri) as inhibitors. Normalized histograms are provided together with the percentages of the stained fractions. In A-C, working protein concentrations are 5 mg/mL each. D) Histograms of dilution series (upper panels) and $IC_{50}$ determination (below) for VP1, HPA, and anti-Forssman IgM M1/87 to ovine blood cells.

**Figure 6: Structural comparison and evolutionary conservation of glycan engagement in polymaviruses. (A)** Six different modes of sialic acid binding are described in polyomaviruses, such as B-lymphotropic polyomavirus (LPyV, 4MBZ), BK polyomavirus (BKPyV, 4MJo), Merkel Cell polyomavirus (MCPyV, 4FMJ), murine polyomavirus (mPyV, 1VPS), New Jersey polyomavirus (NJPyV, 6Y5Y), and *Trichodysplasia* spinulosa-associated polyomavirus (TSPyV, 4U60) (left). The binding mode of Forssman pentaose ($F_P$) extends over a surface area of ShPyV VP1 that encompasses all sialic acid modes but the TSPyV site (middle). 3D depictions of the Symbol Nomenclature for Glycans (SFNG) of the binding modes are provided below, respectively. VP1 can simultaneously bind to FP and the sialylated trisaccharides 6'sialyllatosamine (6'SLN) or 3' sialyllactosamine (3'SLN). Refined 2Fo-Fc electron densities are displayed as blue meshes with a level of 1 $\sigma$ around the ligands (right). **(B)** Evolutionary connection of glycan engagement in betapolyomaviruses. Superposition of $\alpha$-GalNAc recognition mode by ShPyV VP1 to the sialylated complex structure of the early beta-PyV BKPyV VP1 (left). Engagement is mediated by apolar interactions between a surface-oriented methyl group in the terminal sugar unit and a conserved hydrophobic protein cavity (right).

**Figure 7: Common GalNAc glycoconjugates and related antigens.** $R_1$ - Ceramide (Cer), $R_2$ - O-linked glycoprotein, GSL - Glycosphingolipids, HBGA - Histo blood group antigen, Gb3 - Globotriaose, Gb4 - Globotetraose

The sequences show:

SEQ ID NO: 1 >YP_009134726.1 VP1 [Sheep polyomavirus 1]

MAPKKRSLGQPAPVPKLIVKGGIEVLAVRTGPDSITEIEAYLNPRMGQPQNEDFYGFSDNVTVS DDFGSDAPPWKQFPCYSTARISLPMLNQDMTCDTILMWEAISCRTEVMGVNMLTNVHSAQKR VYENDREGTGIGVEGMGYHMFAIGGEPLELQFMVFNHRATYPAEATVIKNPGASSQVFDPNLK GTLTADGVFPVEAWGDPFKNENTRYFGQYTGGTQTPPVLTFTNTQTTILLDENGVGPLCKGD GLFLSCADIVGFFTQHNKKMSFRGLPRYFRVTLRKRVVKNPYPVSHLLNTLFSNMQPTVRGQP MQGEDAQVEEVRVYQGVEGLPGDPDMVRFRDQFGQTNTVVGN

SEQ ID NO: 2 >Recombinant ShPyV VP1 construct

MGSSHHHHHHSSGLVPRGSHMGGIEVLAVRTGPDSITEIEAYLNPRMGQPQNEDFYGFSDNV TVSDDFGSDAPPWKQFPCYSTARISLPMLNQDMTSDTILMWEAISCRTEVMGVNMLTNVHSA QKRVYENDREGTGIGVEGMGYHMFAIGGEPLELQFMVFNHRATYPAEATVIKNPGASSQVFDP NLKGTLTADGVFPVEAWGDPFKNENTRYFGQYTGGTQTPPVLTFTNTQTTILLDENGVGPLC KGDGLFLSCADIVGFFTQHNKKMSFRGLPRYFRVTLRKRVVKN

SEQ ID NO: 3 >core sequence of ShPyV VP1 construct

IEVLAVRTGPDSITEIEAYLNPRMGQPQNEDFYGFSDNVTVSDDFGSDAPPWKQFPCYSTARISL PMLNQDMTSDTILMWEAISCRTEVMGVNMLTNVHSAQKRVYENDREGTGIGVEGMGYHMF AIGGEPLELQFMVFNHRATYPAEATVIKNPGASSQVFDPNLKGTLTADGVFPVEAWGDPFKN ENTRYFGQYTGGTQTPPVLTFTNTQTTILLDENGVGPLCKGDGLFLSCADIVGFFTQHNKKMSF RGLPRYFRVTLRKRVVKN

SEQ ID NO: 4 S95C_fwd:
GCTGAACCAAGACATGACCTGCGATACCATCCTGATGTGGGAGG

SEQ ID NO: 5 S95C_rev:
CCTCCCACATCAGGATGGTATCGCAGGTCATGTCTTGGTTCAGC

SEQ ID NO: 6 T61I_fwd:
GCGATAACGTGATTGTTAGCGACGATTTCG

SEQ ID NO: 7 T61I_rev:
CGAAATCGTCGCTAACAATCACGTTATCGC

SEQ ID NO: 8 Q266F_fwd:
GGGTTTCTTTACCTTTCACAACAAGAAAATGAGC

SEQ ID NO: 9 Q266F_rev:
GCTCATTTTCTTGTTGTGAAAGGTAAAGAAACCC

EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.
The examples show:

**Example 1:** Forssman pentaose is a ligand for the major capsid protein VP1 of sheep polyomavirus

To identify potential attachment factors of sheep polyomavirus (ShPyV), the inventors subjected the recombinantly expressed major capsid protein VP1 to broad spectrum glycan microarray screening of over 900 different carbohydrate structures (Fig. 1A). As the inventors previously described the structural determinants of sialic acid engagement by ShPyV VP1, the inventors expectedly detected responses for several sialylated glycans such as GM1 pentaose, GD3 tetraose, GTic oligosaccharide, and 6'sialyllactosamine, which are, however, relatively weak. Among the four strongest reliable signals in the glycan array, which are depicted in figure 1, two stem from glycans carrying $\alpha \rightarrow 6$ linked sialic acids, which are 9-O-Acetyl-N-acetylneuraminic acid (Neu5,9Ac) and N-glycolyl-neuraminic acid (Neu5Gc).

Apart from the sialoside signals, the inventors observed a surprisingly strong response for the non-sialylated Forssman glycolipid surpassing all other signals by at least one order of magnitude. Among all carbohydrates in the glycan array the Forssman glycolipid is the only probe that features the Forssman disaccharide GalNAc$\alpha \rightarrow$3GalNAc$\beta$. Samples with a sole terminal GalNAc moiety, such as the $\alpha$-glycosidic human A and Tn antigens and the $\beta$-glycosidic P antigen (for structures see Figure 7) show no significant binding (Fig. 1A), which indicates a narrow specificity of VP1 for the Forssman disaccharide epitope. However, the relatively strong signal occurring for the Gal$\beta$-6GalNAc disaccharide suggests that VP1 tolerates O6-modification of $\alpha$-GalNAc.

To assess whether the interaction of ShPyV VP1 to Forssman pentaose ($F_P$), the glycan portion of Forssman glycolipid, also occurs in a more physiologic setting (*i.e.*, in solution), the inventors conducted a saturation-transfer difference (STD) NMR experiment. STD-NMR is a method based on the nuclear Overhauser effect and reveals liquid-phase ligand binding via transfer of saturated excitation from the protein to the binding partner, which is then visualized by the occurrence of ligand resonances in a difference spectrum. Thereby, the protein resonances remain invisible. 59,60 The inventors recorded the STD-NMR resonances from a sample comprising VP1 and the oligosaccharides Forssman pentaose and 6sialyllactose (6'SL) as ligands, for which the inventors also provide reference spectra (Fig. 2B-C). Analysis of the STD-NMR spectrum (Fig. 2A), depicted in the high field from 4.5-1.6 ppm, reveals binding of $F_P$ to VP1 with the majority of $^1$H resonances being transferred. Since excitation transfer heavily falls off with distance, VP1 is expected to contact an extensive portion of $F_P$ during the binding event. In contrast, at around 2 ppm the signal of a third methyl group in the STD-NMR spectrum manifests as the only transferred resonance of 6'SL. This indicates that $F_P$ and 6'SL are recognized by different binding sites of VP1 and do not interfere with each other.

**Example 2:** Crystal structure of ShPyV VP1 liganded with Forssman pentaose reveals recognition via hydrophobic and polar interactions

To characterize the engagement of Forssman pentaose ($F_P$) by ShPyV VP1 at the atomic level, the inventors solved the crystal structure of the complex at 1.92 Å resolution (Table S2). The crystallographic asymmetric unit (ASU) contains two independent VP1 pentamers arranged in a tail-to-tail orientation while leaving the loops on the protein surface accessible. Difference electron density maps calculated with unliganded VP1 coordinates unambiguously reveal a total number of ten bound $F_P$ molecules. The oligosaccharide densities are located on top of each VP1 in a region that corresponds to the outside of the viral capsid (Fig. 3A). All binding sites are identical and comprise residues from the BC1, DE, and HI loops of one VP1 monomer and the BC2 loop of its clockwise neighbor. (Fig. 3C). The linear $F_P$ oligosaccharide adopts a kinked conformation, which is in good agreement with the solution structure of isolated $F_P$ solved by NMR. [61]

$F_P$ is defined, from non-reducing to reducing ends, by the oligosaccharide sequence GalNAc$\alpha^5 \rightarrow$3GalNAc$\beta^4 \rightarrow$3Gal$\alpha^3 \rightarrow$4Gal$\beta^2 \rightarrow$4Glc$^1$. The $\alpha$GalNAc$^5$ of $F_P$ binds to VP1 through hydrophobic van-der-Waals interactions in a surface cavity constituted by phenylalanine residues of the BC1 (F53/56), HI (F264), and the clockwise neighboring BC2 loop (F66cw) (Fig. 3B). This cavity already exists in the unliganded protein structure (see PDB 6Y61) and accommodates the $\alpha$-GalNAc5 methyl group without structural rearrangements. In addition to the nonpolar interactions, $\alpha$GalNAc$^5$ forms hydrogen bonds with the side chain of Q266 and the backbone of Phe66cw and D64cw (Fig. 3B). The subsequent $\beta$GalNAc$^4$ of $F_P$ is rotated 180 degrees around the glycosidic bond with respect to the terminal $\alpha$GalNAc$^5$. Therefore, the methyl and amide groups of $\beta$GalNAc$^4$ project away from the VP1 surface and do not contact the protein. However, the $\beta$GalNAc$^4$ carbonyl oxygen forms hydrogen bonds with the side chains of Q266 and N268. Additional hydrogen bonds to $\beta$GalNAc$^4$ involve the side chains of Q47, N59, and Q266. Below $\beta$GalNAc$^4$, two water molecules bridge the sugar to the hydrophilic surface of VP1 (Fig. 3B). The next monosaccharide in sequence, $\alpha$Gal$^3$, contacts the protein via hydrogen bonds to the side chains of D58, N59, and K270 (Fig. 3C). An indirect, water-mediated interaction occurs between the $O_4$ hydroxyl group of $\alpha$Gal$^3$ and the backbone oxygen of N59 (Fig. 3B). The glycosidic linkage between $\alpha$-Gal$^3$ and $\beta$Gal$^2$ induces a conformational kink in the oligosaccharide and diverts the remaining portion of $F_P$ away from VP1. Therefore, $\beta$Gal$^2$ and the truncal Glc$^1$ do not contact the protein surface.

Due to the costliness of Forssman glycans, conventional biophysical affinity determination techniques were not applied. To still establish an approximate dissociation constant value, the inventors derivatize isomorphous ShPyV VP1 crystals with serially diluted Forssman pentaose ($F_P$) solution and collect the diffraction data. The resulting electron density of $F_P$ is integrated for each data set and plotted as a function of its concentration (Fig. 3C). [16] The inventors analyze the dose response data using a Michaelis-Menten approach and thus approximate the dissociation constant between VP1 and $F_P$ to 38.2 $\mu$M.

The inventors presume that the Forssman disaccharide GalNAc$\alpha$5→3GalNAc4, which promotes the hydrophobic interactions to VP1, is the main determinant for tropism. To corroborate this hypothesis, the inventors derivatized ShPyV VP1 crystals with globo-*N*-tetraose (Gb4), the glycan of the globoseries P antigen and molecular precursor of $F_P$, and determined the structure of the complex (Table S2). Refinement using unliganded VP1 coordinates reveals only weak electron density of Gb4 indicating a negligible binding affinity (Fig. SxA). The inventors thus infer that globoseries glycolipids shorter than Forssman antigen are only recognized transiently by VP1 and do not significantly contribute to target cell recognition.

Additionally, the structural elucidation of the VP1-$F_P$ complex allows for the superposition of the epitopes of other aGalNAc antigens such as A (GalNAc$\alpha$→3(Fuc$\alpha$→2)Gal-R) and Tn (GalNAc$\alpha$→O-Ser/Thr) into the identified Forssman binding site. The resulting models show that the remaining glycan portions of A and Tn antigen would severely clash with the protein surface, rendering binding of these molecules in their established conformations impossible (Fig. SxB). Congruently, derivatives of ShPyV VP1 crystals with A antigen trisaccharide and T antigen (Gal$\beta$→3-Tn) resulted in unliganded VP1 pentamers after structure solution (unpublished results).

**Example 3:** GalNAc5 $\alpha$-face recognition by VP1 confers specificity and defines a novel binding mode for the Forssman disaccharide

In the following the inventors compare the engagement of Forssman pentaose ($F_P$) by ShPyV VP1 with other concurrent Forssman-related entries deposited in the RCSB protein data bank (PDB). Until now, four distinct protein structures from three different aGalNAc lectin classes are available in complex with Forssman glycans, which are human galectin-9 (Gal-9), legume (L-type) lectin *Dolichos biflorus* agglutinin (DBA), and the homologous Htype lectins *Helix pomatia* agglutinin (HPA) and *Sinularia lochmodes* lectin-2 (SLL2).

Monosaccharides, which are glycosidically linked within an oligosaccharide, feature two faces corresponding to the orientation of the anomeric oxygen in the respective $\alpha$- or $\beta$-conformations. This defines that VP1 recognizes Forssman pentaose via the $\alpha$face of the terminal GalNAc5, and due to this specific binding conformation the two subsequent monosaccharides of $F_P$ are facilitated to pass along and closely interact with the protein surface (Fig. 5A).

In contrast, the human Gal-9 carbohydrate recognition domain (CRD) was found to mainly interact with the $\beta$-GalNAc4 of $F_P$. The $\alpha$GalNAc5 orientates towards the Gal-9 CRD surface with the Nacetyl group rather than any of its faces and only contacts the protein directly via its ring and O6 oxygen atoms (Fig. 5B). [62] Within a carbohydrate library screening, Gal-9 displayed the highest affinity towards $F_P$ ($K_D$ = 0.3 $\mu$M) but binding was rather unspecific. It recognizes a variety of different glycan structures with two-digit micromolar dissociation constants, e.g. the $\beta$-GalNAc-terminating $F_P$ precursor molecule globoside ($K_D$ = 25 $\mu$M). [62]

L-type DBA and H-type HPA lectins are unrelated proteins but use convergent strategies to engage terminal aGalNAc. In the Forssman glycan complexes, both lectins recognize the $\beta$-face of $\alpha$GalNAc5 in binding sites that tolerate variable subsequent monosaccharides with a limited number of contacts (Fig. 5C-D). [63-65] Consequently, HPA was found to engage, with decreasing affinity, the glycans of Forssman antigen (GalNAc$\alpha$→3GalNAc$\beta$-R), blood group A antigen (GalNAc$\alpha$→3(Fuc$\alpha$→2)Gal$\beta$-R), and Tn antigen (GalNAc$\alpha$→O-Ser/Thr). [66] Collectively, the inventors propose that the Forssman recognition via the $\alpha$-face of $\alpha$-GalNAc5 by VP1 is unprecedented among the established lectins for Forssman glycans and thus defines a novel engagement strategy for this group of oligosaccharides.

**Example 4:** ShPyV VP1 specifically attaches to Forssman-positive cells *in vitro*

To unveil the importance of glycan engagement for cell attachment by sheep polyomavirus (ShPyV), the inventors designed structurebased mutants of its VP1 and subjected them to flow cytometry analysis of donor animal blood cells from sheep (Forssman-positive, FORS+) and cattle (Forssman-negative, FORS-). Two independent single amino acid substitutions were introduced in the binding sites of the distinct glycoconjugate classes, i.e., Forssman antigen (FA) and sialoside receptors. As positive and negative controls the inventors included the $\alpha$-*N*-acetylgalactosamine (aGalNAc) lectin *Helix pomatia* agglutinin (HPA) and the VP1 of the sialoside but non-Forssman binding human *Trichodysplasia* spinulosa-associated polyomavirus (TSPyV) and its established sialic acid site mutant, respectively. [15] Similar to HPA, binding to sheep cells is saturated for the wild type (wt) and the sialic acid site mutant T61I of ShPyV VP1. In contrast, introduction of Q266F into the FA site of ShPyV VP1 essentially abolishes FORS+ cell binding (Fig. 5A). In contrast, weak sialoside-based binding to FORS-bovine blood cells occurs for the wt VP1 of both ShPyV and TSPyV and is reduced in the respective sialic acid site mutants (Fig. 5A). These results suggest that Forssman antigen is the main determinant for the high fidelity FORS+ cell tropism by ShPyV VP1 rather than sialylated glycoconjugates.

The inventors also examine the engagement of FORS- human erythrocytes by ShPyV VP1 and compare the results to HPA, which is a commonly used indicator for aberrant glycosylation in cancerogenesis. HPA uses a single binding site for the recognition of terminal aGalNAc epitopes in the tumor-associated Forssman and Tn antigens but also binds to blood group A antigen. [64,66-76] The inventors show that VP1 does not attach to any FORS- human blood cells from blood types A1, A2, B, and O, while HPA is strongly retained by A1 and more mildly by A2 cells (Fig. 5B). Incubation of VP1 with an excess of Forssman triaose (F-tri) inhibits binding to FORS+ sheep blood cells but not with A antigen triaose (A-tri). In contrast, FORS+ cell binding by HPA is inhibited by exceeding amounts of both F-tri and A-tri (Fig. 5C).

To quantify the FORS+ cell engagement, the inventors collect dose response data of decreasing VP1 concentrations in the presence of constant numbers of sheep blood cells and compared them to HPA and the monoclonal anti-Forssman IgM M1/87. Data analysis results in an IC50 value of 0.19 $\mu$g/mL for VP1, a value that is comparable to HPA (0.41 $\mu$g/mL) and one order of magnitude lower than M1/87 (3 $\mu$g/mL).

**Example 5:** Evolutionary analysis of different VP1 reveals conserved glycan engagement strategies in betapolyomaviruses

Previously, the inventors have shown that ShPyV VP1 shares a conserved sialic acid binding site with the major capsid proteins of selected alphapolyomaviruses such as Trichodysplasie spinulosa-associated Polyomavirus (TSPyV) and gammapolyomaviruses such as Ghoose hemorrhagic and Finch Polyomavirus (GhPyV and FiPyV, respectively). [19] In this paragraph, the inventors briefly compare the recognition of Forssman pentaose in ShPyV VP1 to the established strategies for glycan engagement in other polyomaviruses and explore the underlying evolutionary connections.

So far, the major capsid proteins of 13 different polyomaviruses have structurally been shown to bind to sialylated glycans, thereby revealing not less than six different modes for the specific recognition of sialic acids (Fig. 6A, left). [12,14-17,19] Superposition of the novel Forssman pentaose ($F_P$) binding mode by sheep polyomavirus VP1 and these Neu5Ac structures shows that upon binding, the $F_P$ oligosaccharide engages an area of ShPyV VP1 that comprises five out of six previously described sialic acid modes in other VP1, except for the designated TSPyV site (Fig. 6A, middle). Consequently, the inventors solved additional structures of ShPyV VP1 crystals derivatized with $F_P$ and 6'sialyllatosamine (6'SLN) or 3' sialyllactosamine (3'SLN) (Table S2). These structures unambiguously show that VP1 simultaneously engages both glycan classes with no close contact between them and that the binding of one compound does not affect the binding or conformation of the other (Fig. 6A, right).

Sheep polyomavirus (ShPyV) is a betapolyomavirus only distantly related to the more renowned betapolyomavirus species of simian and human origin such as SV40, BKPyV, and JCPyV (designated "early beta-PyVs"). [31,33] Yet, from superposition of the ShPyV VP1-$F_P$ binding mode onto the BKPyV VP1-GD3 complex structure (PDB 4MJo) the inventors observe that the recognition of sialic acids by BKPyV and the aGalNAc of Forssman by ShPyV rely on fundamentally similar principles (Fig 6B, left). Both VP1 recognize their glycan ligands via the N-acetyl's methyl group in the terminal monosaccharide, which in both cases orientates towards a hydrophobic cavity at the bottom of a shallow depression in the surface of these proteins. And although the residues for the specific recognition of sialic acids in the early PyVs are not present in ShPyV VP1, multiple sequence alignment of their VP1 reveals that the hydrophobic cavity is indeed conserved (Fig. 6B, right). An analogous scenario was reported for the VP1 of African green monkey B-lymphotropic polyomavirus (LPyV) and human polyomavirus 9, which compared to BKPyV utilize an aligned but more recessed hydrophobic surface pocket to bind sialic acids. [14,18] The inventors consider an ancient carbohydrate engagement strategy to be the basis for divergent evolution towards the recognition of different monosaccharides in this specific region of VP1. For ShPyV VP1 it is conceivable, that the primal ability to engage sialic acid in this surface area may have been lost in exchange for the recognition of aGalNAc in Forssman glycans.

After exploring the glycan recognition principles utilized by polyomaviruses of Forssman-negative simian und human origin, the inventors analyzed the VP1 sequences of a number of polyomaviruses deriving from Forssman-positive hosts, such as horse, house mouse, wild goat, and dog. Among these viruses, only murine polyomavirus 2 (mPyV2) indicates Forssman site conservation in its VP1. Interestingly, mPyV2 derives from a clade of betapolyomaviruses sharing a common root with ShPyV. It is part of a monophyletic virus group (here designated "later beta-PyVs") hosted by bats, murids and also African lion. [33,77] Similar to mPyV2, most members of this group show a high degree of conservation in the residues the inventors found essential for Forssman recognition. In contrast, no such conservation can be found in the structurally characterized members of the alpha-, and gammapolyomavirus families. Recapitulative, the ability to engage Forssman glycans seems to manifest exclusively in betapolyomaviruses and is likely an evolutionary result, which may have allowed for past host switching events.

**Materials and methods**

Protein expression and purification

Protocols for the recombinant production of sheep and *Trichodysplasia-spinulosa* polyomavirus VP1 are available. [15,19] Briefly, transformed *E. coli* BL21 (DE3) were grown in LB (Miller) medium supplemented with 100 $\mu$g/ml ampicillin at 37 °C. Expression was induced at an $OD_{600}$ of 0.7 with 400 $\mu$M IPTG and carried out at 20 °C for 16 h. VP1 were extracted from bacterial pellets by sonication and subsequent centrifugation (17,000 rpm at 4 °C). The $His_6$-tagged VP1 were purified by immobilized-metal affinity chromatography (IMAC) and subsequent gel filtration (20 mM HEPES pH 7.5, 150 mM NaCl). For crystallization of ShPyV VP1 the $His_6$-tag was removed by incubation with 10 U/mg thrombin at room temperature overnight. Cleaved tags and thrombin were removed by IMAC and gel filtration. If not immediately used, VP1 were stored at 20 °C.

NMR

All nuclear magnetic resonance (NMR) data were collected in a Bruker AVIII-600 MHz spectrometer equipped with a TXI-z probe head at 298 K. The inventors performed the experiments using 3 mm NMR tubes. Samples were provided in 99 % $D_2O$-PBS containing 20 mM $KH_2PO_4/K_2HPO_4$ (pH 7.4) and 150 mM NaCl. Initially, $^1$H reference spectra of isolate oligosaccharides samples (4 mM Forssman pentaose and 2 mM 6'SL, respectively) were recorded. Subsequently, the $^1$H resonances of a sample comprising 1 mM Forssman pentaose and 1 mM 6'SL in presence of 20 $\mu$M ShPyV VP1 were collected. From the same sample, the saturation difference transfer (STD) NMR experiment was performed. All pulse sequences were set up according to the previously described protocol. [19] NMR spectra were processed and displayed with TOPSPIN 4 (Bruker). All carbohydrates were purchased from Carbosynth, UK.

Crystallization and data collection

Purified ShPyV VP1 was concentrated to 3.5 mg/ml. Crystallization droplets were set up comprising 1 $\mu$l protein and 1 $\mu$l reservoir solution (150 mM KSCN and 20 % (w/v) PEG 3,350). The droplets were equilibrated against the 500 $\mu$l reservoir using the hanging drop vapor diffusion method at 20 °C. For ligand derivatization, VP1 crystals were soaked in crystallization solution supplemented with the respective oligosaccharides (10 mM FA pentaose, 10 mM globo-N-tetraose, 5 mM FA pentaose + 20 mM 3'SLN, and 5 mM pentaose + 20 mM 6'SLN), at 20 °C for half an hour. Prior to flash freezing, crystals were transferred to soaking solutions supplemented with 20 % (v/v) MPD for cryo-protection. X-ray diffraction experiments were performed using the Swiss Light Source (SLS) beamline X06DA at the PSI (Villigen, Switzerland). Data were collected at a wavelength of 1 Å and an excitation time and angle of 0.1 s and 0.1° per image, respectively. All carbohydrates were purchased from Carbosynth, UK.

Structure determination and refinement

X-ray diffraction images were integrated and scaled using XDS software. [73] For each data set, 2000-3000 reflections were reserved as a test set for structure validation. Phases were determined by molecular replacement using the native sheep polyomavirus VP1 structure as search model (PDB ID 6Y61) in PHASER (CCP4). [74] Structure refinement was performed using Phenix (simulated annealing, reciprocal space, real-space, B-factor, and occupancy refinement) and CCP4 Refmac5 (restrained maximum-likelihood refinement). [75,76] Manual structure building was done in Coot. [77] Oligosaccharide models were built according to the conventional monosaccharide restraints from the CCP4 libraries. Alternating rounds of refinement and model building were performed until validation parameters converged.

Crystallographic affinity determination

To approximate the affinity towards FA pentaose ($F_P$) the inventors prepared a glycan dilution series in VP1 crystals, an approach that was successfully utilized in the past. [16,23] VP1 crystals were grown as described above. The reference soaking solution (10 mM $F_P$ in 150 mM KSCN, 20 % (w/v) PEG 3,350 and 20 % (v/v) MPD) was diluted using 1:2 steps down to a final concentration of 0.0195 mM. Distinct crystals and equal soaking times were used for the parallel derivatization with $F_P$ at the different concentrations. Data collection was performed as described above. For processing, the unit cell constants of all data sets were treated as isomorphous. All diffraction intensities were scaled against the 10 mM $F_P$ reference data set using scaleit. [78] A single structure solution was sufficient for all data sets. A VP1 model lacking all water and carbohydrate atoms, and a control tripeptide (AA 143-145) in all chains, was used for simulated annealing refinement (Phenix). [75] The bias-reduced positive Fo-Fc electron densities were integrated within 1Å around the terminal three residues of the refined $F_P$ model and the control tripeptide, respectively, for all individual VP1 chains in all data sets (Mapman). [79] The electron density integrals were plotted against the ligand concentrations using R software environment. A Michaelis-Menten curve (R library "drc") was fit to the data using the equation:

$$y = b_{max} * \frac{x}{(a+x)}$$

The integrated electron densities (y) are a function of the ligand concentration x. The parameters $\alpha$ and $b_{max}$ represent the crystallographic affinity and the asymptote of the density integrals, respectively.

VP1 mutagenesis and labeling

Site-directed mutagenesis of VP1 was performed within the pET-15b plasmid DNA. Reaction samples of 20 μl contained 1× ReproFast amplification buffer (Genaxxon), 20 ng template DNA, 50 ng primers, 2 mM dNTP, and 1 unit of ReproFast polymerase (Genaxxon). Initial denaturation was performed at 95 °C for 2 minutes, followed by 18 cycles of 95 °C for 1 minute (denaturation), 55-70 °C gradient for 1 minute (annealing), and 72 °C for 6 minutes (synthesis). Subsequently, synthesis was completed at 72 °C for 10 minutes. Samples comprising the same primer pairs were pooled. Parental template DNA was digested with 2 units of DpnI at 37 °C for two hours. The resulting PCR product was directly transformed into *E. coli* DH5α. DNA sequencing was performing using the Sanger method (Microsynth Seqlab).
DNA primers (ThermoFisher Scientific) displayed from 5'- to 3'.

S95C_fwd:    GCTGAACCAAGACATGACCTGCGATACCATCCTGATGTGGGAGG

S95C_rev:    CCTCCCACATCAGGATGGTATCGCAGGTCATGTCTTGGTTCAGC

T61I_fwd:    GCGATAACGTGATTGTTAGCGACGATTTCG

T61I_rev:    CGAAATCGTCGCTAACAATCACGTTATCGC

Q266F_fwd:   GGGTTTCTTTACCTTTCACAACAAGAAAATGAGC

Q266F_rev:   GCTCATTTTCTTGTTGTGAAAGGTAAAGAAACCC [DK1]

For fluorescence labeling, VP1 was expressed and purified like described above. Surficial cysteine residues were reduced in degassed SEC buffer (20 mM HEPES pH 7.4, 150 mM NaCl) supplemented with 80 mM DTT at 4 °C overnight. DTT was removed from the protein solution using a PD-10 desalting column (Cytiva) with DTT-less SEC buffer. A 20fold molar excess of cysteine reactive AFDye 488 Maleimide (Fluoroprobes) was added to the protein solution (0.5 - 0.7 mg/ml) and the reaction was incubated in the dark at 4 °C for 16-20 h. Excess dye was quenched and removed by desalting in SEC buffer supplemented with 10 mM DTT. VP1-488 conjugate was eluted in the same buffer. The degree of labeling was photometrically determined according to the manufacturer's protocol.

Flow cytometry

Erythrocytes from defibrinated blood were diluted into FACS buffer (1× PBS + 2 % FCS) to a density of $10 \times 10^6$ cells / mL. Samples of $1 \times 10^6$ cells were incubated with labeled protein at room temperature for 30 minutes with occasional shaking. Samples were then directly transferred to the flow cytometer (LSR II, BD Biosciences). Singlet cells were gated and the fluorescent population was captured at 520 nM. M1/87-FITC (Santa Cruz Biotechnology) and HPA Alexa Fluor™ 488 conjugate (Invitrogen), respectively were used as references. Defibrinated sheep and bovine blood were purchased from TCS Biosciences Ltd and BioTrading Benelux B.V., respectively.

Data availability

Structure models of ShPyV VP1 with its carbohydrate ligands were deposited on the RCSB protein data bank (PDB) with the accession codes 7B6S (FA pentaose), 7B6T (globo-N-tetraose), 7B6U (FA pentaose + 6'SLN), and 7B6V (FA pentaose + 3'SLN).

REFERENCES

The references are:

1. Cheng, J., DeCaprio, J. A., Fluck, M. M. & Schaffhausen, B. S. Cellular transformation by Simian Virus 40 and Murine Polyoma Virus T antigens. Seminars in Cancer Biology (2009) doi:10.1016/j.semcancer.2009.03.002.

2. Feng, H., Shuda, M., Chang, Y. & Moore, P. S. Clonal integration of a polyomavirus in human Merkel cell carcinoma. Science (80-. ). 319, 1096-1100 (2008).

3. Chang, Y. & Moore, P. S. Merkel cell carcinoma: A virus-induced human cancer. Annual Review of Pathology: Mechanisms of Disease (2012) doi:10.1146/annurev-pathol-011110-130227.

4. Salunke, D. M., Caspar, D. L. D. & Garcea, R. L. Self-assembly of purified polyomavirus capsid protein VP1. Cell (1986) doi:10.1016/0092-8674(86)90071-1.

5. Barouch, D. H. & Harrison, S. C. Interactions among the major and minor coat proteins of polyomavirus. J. Virol. 68, 3982-3989 (1994).

6. Chen, X. S., Stehle, T. & Harrison, S. C. Interaction of polyomavirus internal protein VP2 with the major capsid protein VP1 and implications for participation of VP2 in viral entry. EMBO J. 17, 3233-3240 (1998).

7. Tsai, B. et al. Gangliosides are receptors for murine polyoma virus and SV40. EMBO J. 22, 4346-4355 (2003).

8. Smith, A. E., Lilie, H. & Helenius, A. Ganglioside-dependent cell attachment and endocytosis of murine polyomavirus-like particles. FEBS Lett. (2003) doi:10.1016/S0014-5793(03)01220-1.

9. Erickson, K. D., Garcea, R. L. & Tsai, B. Ganglioside GTib Is a Putative Host Cell Receptor for the Merkel Cell Polyomavirus. J. Virol. 83, 10275-10279 (2009).

10. Gilbert, J. et al. Ganglioside GD1a Restores Infectibility to Mouse Cells Lacking Functional Receptors for Polyomavirus. J. Virol. 79, 615-618 (2005).

11. Blaum, B. S. & Stehle, T. Sialic Acids in Nonenveloped Virus Infections. in Advances in Carbohydrate Chemistry and Biochemistry vol. 76 65-111 (2019).

12. Neu, U. et al. Structures of merkel cell polyomavirus VP1 complexes define a sialic acid binding site required for infection. PLoS Pathog. 8, 8 (2012).

13. Neu, U. et al. A Structure-Guided Mutation in the Major Capsid Protein Retargets BK Polyomavirus. PLoS Pathog. 9, 01003688 (2013).

14. Neu, U. et al. Structures of B-Lymphotropic Polyomavirus VP1 in Complex with Oligosaccharide Ligands. PLoS Pathog. 9, (2013).

15. Ströh, L. J. et al. Trichodysplasia spinulosa-Associated Polyomavirus Uses a Displaced Binding Site on VP1 to Engage Sialylated Glycolipids. PLoS Pathog. 11, (2015).

16. Stehle, T. & Harrison, S. C. Crystal structures of murine polyomavirus in complex with straight-chain and branched-chain sialyloligosaccharide receptor fragments. Structure 4, 183-194 (1996).

17. Neu, U., Woellner, K., Gauglitz, G. & Stehle, T. Structural basis of GM1 ganglioside recognition by simian virus 40. Proc. Natl. Acad. Sci. U. S. A. 105, 5219-5224 (2008).

18. Khan, Z. M. et al. Crystallographic and Glycan Microarray Analysis of Human Polyomavirus 9 VP1 Identifies N-Glycolyl Neuraminic Acid as a Receptor Candidate. J. Virol. 88, 6100-6111 (2014).

19. Ströh, L. J. et al. Structural basis and evolution of glycan receptor specificities within the polyomavirus family. MBio 11,1-21 (2020).

20. O'Hara, S. D., Stehle, T. & Garcea, R. Glycan receptors of the Polyomaviridae: Structure, function, and pathogenesis. Current Opinion in Virology vol. 7 73-78 (2014).

21. Stehle, T. & Khan, Z. M. Rules and Exceptions: Sialic Acid Variants and Their Role in Determining Viral Tropism. J. Virol. 88, 7696-7699 (2014).

22. Stehle, T., Yan, Y., Benjamin, T. L. & Harrison, S. C. Structure of murine polyomavirus complexed with an oligosaccharide receptor fragment. Nature 369,160-163 (1994).

23. Stehle, T., Gamblin, S. J., Yan, Y. & Harrison, S. C. The structure of simian virus 40 refined at 3.1 Å resolution. Structure 4,165-182 (1996).

24. Rustmeier, N. H., Strebl, M. & Stehle, T. The symmetry of viral sialic acid binding sites-implications for antiviral strategies. Viruses vol. 11 947 (2019).

25. Elphick, G. F. et al. The human polyomavirus, JCV, uses serotonin receptors to infect cells. Science (80-.). 306, 1380-1383 (2004).

26. Assetta, B. et al. 5-HT2 Receptors Facilitate JC Polyomavirus Entry. J. Virol. 87, 13490 (2013).

27. Schowalter, R. M., Pastrana, D. V. & Buck, C. B. Glycosaminoglycans and sialylated glycans sequentially facilitate merkel cell polyomavirus infectious entry. PLoS Pathog. 7, 1002161 (2011).

28. Hurdiss, D. L., Frank, M., Snowden, J. S., Macdonald, A. & Ranson, N. A. The Structure of an Infectious Human Polyomavirus and Its Interactions with Cellular Receptors. Structure 26, 839 (2018).

29. Geoghegan, E. M. et al. Infectious Entry and Neutralization of Pathogenic JC Polyomaviruses. Cell Rep. 21, 1169-1179 (2017).

30. Assetta, B. et al. Genetic and Functional Dissection of the Role of Individual 5-HT2 Receptors as Entry Receptors for JC Polyomavirus. Cell Rep. 27, 1960-1966.06 (2019).

31. Buck, C. B. et al. The Ancient Evolutionary History of Polyomaviruses. PLoS Pathog. 12, 1005574 (2016).

32. Calvignac-Spencer, S. et al. A taxonomy update for the family Polyomaviridae. Arch. Virol. 161, 1739-1750 (2016).

33. Torres, C. Evolution and molecular epidemiology of polyomaviruses. Infection, Genetics and Evolution vol. 79 104150 (2020).

34. Varki, A. et al. Essentials of Glycobiology. Cold Spring Harb. 823 (2017).

35. Sonnenberg, A. et al. Monoclonal antibodies detecting different epitopes on the Forssman glycolipid hapten. J. Immunol. (1986).

36. Haslam, D. B., Baenziger, J. U., Baenzigert, J. U. & Baenziger, J. U. Expression cloning of Forssman glycolipid synthetase: A novel member of the histo-blood group ABO gene family. Proc. Natl. Acad. Sci. U. S. A. 93, 10697-10702 (1996).

37. Yamamoto, F. et al. An integrative evolution theory of histo-blood group ABO and related genes. Sci. Rep. 4, 6601 (2014).

38. Yamamoto, M., Cid, E. & Yamamoto, F. Molecular genetic basis of the human Forssman glycolipid antigen negativity. Sci. Rep. 2, 975 (2012).

39. Tanaka, N. & Leduc, E. H. A Study of the Cellular Distribution of Forssman Antigen in Various Species. J. Immunol. 77, 198-212 (1956).

40. Stromberg, N. et al. Host-specificity of uropathogenic Escherichia coli depends on differences in binding specificity to Gal$\alpha$1-4Gal-containing isoreceptors. EMBO J. 9, 2001-2010 (1990).

41. Stromberg, N., Nyholm, P. G., Pascher, I. & Normark, S. Saccharide orientation at the cell surface affects glycolipid receptor function. Proc. Natl. Acad. Sci. U. S. A. 88, 9340-9344 (1991).

42. Johanson, I., Lindstedt, R. & Svanborg, C. Roles of the pap- and prs-encoded adhesins in Escherichia coli adherence to human uroepithelial cells. Infection and Immunity vol. 60 3416-3422 (1992).

43. Lanne, B. et al. Glycoconjugate Receptors for P-fimbriated Escherichia coli in the Mouse: AN ANIMAL MODEL OF URINARY TRACT INFECTION *. J. Biol. Chem. 270, 9017-9025 (1995).

44. Müthing, J. et al. Promiscuous Shiga toxin 2e and its intimate relationship to Forssman. Glycobiology 22, 849-862 (2012).

45. Elliott, S. P., Yu, M., Xu, H. & Haslam, D. B. Forssman Synthetase Expression Results in Diminished Shiga Toxin Susceptibility: A Role for Glycolipids in Determining Host-Microbe Interactions. Infect. Immun. 71, 6543-6552 (2003).

46. Young, W. W., Hakomori, S.-I. & Levine, P. Characterization of Anti-Forssman (ANTI-Fs) Antibodies in Human Sera: Their Specificity and Possible Changes in Patients with Cancer. 123, (1979).

47. Svensson, L. et al. Forssman expression on human erythrocytes: Biochemical and genetic evidence of a new histo-blood group system. Blood 121, 1459-1468 (2013).

48. Kawanami, J. The Appearance of Forssman Hapten in Human Tumor. Journal of Biochemistry vol. 72 783-785 (Oxford University Press, 1972).

49. Hakomori, S., Wang, S. M. & Young, W. W. Isoantigenic expression of Forssman glycolipid in human gastric and colonic mucosa: Its possible identity with 'A like antigen' in human cancer. Proc. Natl. Acad. Sci. U. S. A. 74, 3023-3027 (1977).

50. Yoda, Y., Ishibashi, T. & Makita, A. Isolation, Characterization, and Biosynthesis of Forssman Antigen in Human Lung and Lung Carcinoma. J. Biochem. 88, 1887-1894 (1980).

51. Taniguchi, N., Yokosawa, N., Narita, M., Mitsuyama, T. & Makita, A. Expression of forssman antigen synthesis and degradation in human lung cancer. J. Natl. Cancer Inst. 67, 577-583 (1981).

52. Mori, T. et al. Forssman antibody levels in sera of cancer patients. Immunol. Invest. 11, 217-225 (1982).

53. Mori, T., Sudo, T. & Kano, K. Expression of Heterophil Forssman Antigen on Cultured Malignant Cell Lines. JNCI J. Natl. Cancer Inst. 70, 811-818 (1983).

54. Hakomori, S. Tumor-Associated Carbohydrate Antigens. Annual review of immunology vol. 2 103-126 (Annual Reviews 4139 El Camino Way, P.O. Box 10139, Palo Alto, CA 94303-0139, USA , 1984).

55. Uemura, K., Hattori, H., Ono, K., Ogata, H. & Taketomi, T. Expression of Forssman glycolipid and blood group-related antigens A, Le(x) and Le(y) in human gastric cancer and in fetal tissues. Jpn. J. Exp. Med. 59, 239-249 (1989).

56. Ono, K. et al. Expression of Forssman antigen in human large intestine. J. Histochem. Cytochem. 42, 659-665 (1994).

57. Cid, E., Yamamoto, M. & Yamamoto, F. Mixed-Up Sugars: Glycosyltransferase Cross-Reactivity in Cancerous Tissues and Their Therapeutic Targeting. ChemBioChem (2021) C101:10.1002/cbic.202100460.

58. Hirayama, R. et al. Changes in Serum Levels of Forssman-Like Antibody in Patients With Gastric Cancer. Cancer 63, 1528-1528 (1989).

59. Mayer, M. & Meyer, B. Group epitope mapping by saturation transfer difference NMR to identify segments of a ligand in direct contact with a protein receptor. J. Am. Chem. Soc. (2001) doi:10.102/jao100120.

60. Blaum, B. S., Neu, U., Peters, T. & Stehle, T. Spin ballet for sweet encounters: Saturation-transfer difference NMR and X-ray crystallography complement each other in the elucidation of protein-glycan interactions. Acta Crystallographica Section F: Structural Biology Communications vol. 74 451-462 (2018).

61. Grönberg, G., Nilsson, U., Bock, K. & Magnusson, G. Nuclear magnetic resonance and conformational investigations of the pentasaccharide of the Forssman antigen and overlapping di-, tri-, and tetra-saccharide sequences. Carbohydr. Res. 257, 35-54 (1994).

62. Nagae, M. et al. Structural Analysis of the Human Galectin-9 N-terminal Carbohydrate Recognition Domain Reveals Unexpected Properties that Differ from the Mouse Orthologue. J. Mol. Biol. 375, 119-135 (2008).

63. Hamelryck, T. W. et al. Carbohydrate binding, quaternary structure and a novel hydrophobic binding site in two legume lectin oligomers from Dolichos biflorus. J. Mol. Biol. 286, 1161-1177 (1999).

64. Lescar, J. et al. Structural basis for recognition of breast and colon cancer epitopes Tn antigen and Forssman disaccharide by Helix pomatia lectin. Glycobiology 17,1077-1083 (2007).

65. Kita, A. et al. Crystal structure of octocoral lectin SLL-2 complexed with Forssman antigen tetrasaccharide. Glycobiology 27, 696-700 (2017).

66. Wu, A. M. & Sugii, S. Coding and classification of d-galactose, N-acetyl-d-galactosamine, and $\beta$-d-Galp-[1$\rightarrow$(4)]-$\beta$-d-GlcpNAc, specificities of applied lectins. Carbohydr. Res. 213, 127-143 (1991).

67. Rambaruth, N. D., Greenwell, P. & Dwek, M. V. The lectin Helix pomatia agglutinin recognizes O-GlcNAc containing glycoproteins in human breast cancer. Glycobiology 22, 839-848 (2012).

68. Parameswaran, R. et al. Binding of aberrant glycoproteins recognizable by Helix pomatia agglutinin in adrenal cancers. BJS Open 2, 353-359 (2018).

69. Jimbo, M. et al. Possible involvement of glycolipids in lectin-mediated cellular transformation of symbiotic microalgae in corals. J. Exp. Mar. Bio. Ecol. 439,129-135 (2013).

70. Hammarstrom, S., Kabat, E. A., Hammarstromf, S. & Kabat, E. A. Purification and Characterization of a Blood-Group A Reactive Hemagglutinin from the Snail Helix pomatia and a Study of Its Combining Site*. Biochemistry 8, 2696-2705 (1969).

71. Torres, B. V. & Smith, D. F. Purification of Forssman and human blood group A glycolipids by affinity chromatography on immobilized Helix pomatia lectin. Anal. Biochem. 170, 209-219 (1988).

72. Schumacher, U. et al. Helix Pomatia Agglutinin Binding Is a Useful Prognostic Indicator in Colorectal Carcinoma. Cancer 74, 3104-3107 (1994).

73. Schumacher, U., Adam, E., Brooks, S. A. & Leathem, A. J. Lectin-binding properties of human breast cancer cell lines and human milk with particular reference to Helix pomatia agglutinin. J. Histochem. Cytochem. 43, 275-281 (1995).

74. Schumacher, U. The use of the lectin Helix pomatia agglutinin (HPA) as a prognostic indicator and as a tool in cancer research. Histol. Histopathol. 14, 217-226 (1999).

75. Dwek, M. V. et al. Helix pomatia agglutinin lectin-binding oligosaccharides of aggressive breast cancer. Int. J. Cancer (Pred. Oncol 95, 79-85 (2001).

76. Sanchez, J. F. et al. Biochemical and Structural Analysis of Helix pomatia Agglutinin: A HEXAMERIC LECTIN WITH A NOVEL FOLD. J. Biol. Chem. 281, 20171-20180 (2006).

77. Ehlers, B. et al. Novel Polyomaviruses in Mammals from Multiple Orders and Reassessment of Polyomavirus Evolution and Taxonomy. Viruses 11, 930 (2019).

78. Neu, U., Bauer, J. & Stehle, T. Viruses and sialic acids: Rules of engagement. Current Opinion in Structural Biology vol. 21 610-618 (2011).

79. Sauter, N. K. et al. Hemagglutinins from Two Influenza Virus Variants Bind to Sialic Acid Derivatives with Millimolar Dissociation Constants: A 500-MHz Proton Nuclear Magnetic Resonance Study. Biochemistry 28, 8388-8396 (1989).

80. Burger, M. M. Forssman Antigen exposed on Surface Membrane after Viral Transformation. Nat. New Biol. 1971 23121 231, 125-126 (1971).

81. KARJALAINEN, H. E. & MÄNTYJÄRVI, R. A. FORSSMAN ANTIGEN IN BK VIRUS-INDUCED TUMOR CELL LINES. Acta Pathol. Microbiol. Scand. Sect. C Immunol. 89C, 49-54 (1981).

82. Ju, T., Otto, V. I. & Cummings, R. D. The Tn Antigen-Structural Simplicity and Biological Complexity. Angew. Chemie Int. Ed. 50,1770-1791 (2011).

83. Uhlenbruck, G. & Prokop, O. An Agglutinin from Helix Pomatia,which Reacts with Terminal N-Acetyl-D-Galactosamine. Vox Sang. 11, 519-520 (1966).

84. Stelos, P., Graves Taliaferro, L. & D, P. A. Comparative Study of Rabbit Hemolysins to Various Antigens: III. Chromatographic Analysis of Forssman Hemolysins Induced by Various Antigens. Source J. Infect. Dis. 108, 113-119 (1961).

85. Kabsch, W. et al. XDS. Acta Crystallogr. Sect. D Biol. Crystallogr. (2010).

86. McCoy, A. J. et al. Phaser crystallographic software. J. Appl. Crystallogr. 40, 658-674 (2007).

87. Liebschner, D. et al. Macromolecular structure determination using X-rays, neutrons and electrons: Recent developments in Phenix. Acta Crystallogr. Sect. D Struct. Biol. (2019) doi:10.1107/S2059798319011471.

88. Murshudov, G. N. et al. REFMAC5 for the refinement of macromolecular crystal structures. Acta Crystallogr. Sect. D Biol. Crystallogr. (2011) doi:10.1107/S0907444911001314.

89. Emsley, P. & Cowtan, K. Coot: Model-building tools for molecular graphics. Acta Crystallogr. Sect. D Biol. Crystallogr. (2004) doi:10.1107/S0907444904019158.

90. Buch, M. H. C. C. et al. Structural and Functional Analysis of Murine Polyomavirus Capsid Proteins Establish the Determinants of Ligand Recognition and Pathogenicity. PLoS Pathog. 11, 01005104 (2015).

91. Howell, P. L. Identification of heavy-atom derivatives by normal probability methods. J. Appl. Crystallogr. 25, 81-86 (1992).

92. Kleywegt, G. J. & Jones, T. A. xdlMAPMAN and xdlDATAMAN - Programs for reformatting, analysis and manipulation of biomacromolecular electron-density maps and reflection data sets. Acta Crystallogr. Sect. D Biol. Crystallogr. 52, 826-828 (1996).

**Claims**

1. **A method for the detection of a target oligosaccharide antigen in a biological sample,** the method comprising the steps of:

   (a) Providing the biological sample suspected to contain the target oligosaccharide antigen and an antigen detection component, wherein the antigen detection component comprises:

   i) a major capsid protein (VP1) of sheep polyomavirus (ShPyV), or an antigen binding fragment thereof, or
   ii) a derivative of ShPyV-VP1 having an amino acid sequence which is at least 80% identical to the amino acid sequence of ShPyV-VP1 (SEQ ID NO:1), or an antigen binding fragment of the derivative of ShPyV-VP1;

   (b) Bringing into contact the biological sample and the antigen detection component under conditions allowing for a specific binding between the target oligosaccharide antigen and the antigen detection component;
   (c) Optionally; removal of unbound sample material,
   (d) Detection of a binding between the target oligosaccharide antigen to the antigen detection component; wherein a detected specific binding in (d) indicates the presence of the target oligosaccharide antigen in the sample;
   *characterized in that* the target oligosaccharide antigen comprises at least a disaccharide GalNAc$\alpha$5→3GalNAc$\beta$4.

2. The method of claim 1, wherein the target oligosaccharide antigen comprises the Forssman pentose.

3. The method of claim 1 or 2, wherein the binding between the target oligosaccharide antigen to the antigen detection component is detected by saturation-transfer difference (STD) NMR, or by using a detectable moiety.

4. The method of any one of claims 1 to 3, wherein the antigen detection component is soluble or is bound to a solid matrix, such as an array or bead.

5. **An *in-vitro* method for the detection of an expression of the Forssman antigen in a human subject,** the method comprising providing a biological sample of the human subject, and performing with the biological sample of the human subject the method for detection of a target oligosaccharide antigen in a biological sample according to any one of claims 1 to 4, and wherein the target oligosaccharide antigen is the Forssman antigen.

6. The *in-vitro* method of claim 5, wherein the subject is a healthy subject, and the method is for a determination of the FORS1+ blood type in the human subject.

7. The *in-vitro* method of claim 5, wherein the biological sample is a blood sample.

8. **An *in-vitro* method of diagnosing a pathological condition in a human subject,** the method comprising the steps of: providing a biological sample of the human subject, and performing with the biological sample of the human subject the method for detection of a target oligosaccharide antigen in a biological sample according to any one of claims 1 to 4, and wherein the target oligosaccharide antigen is the Forssman antigen, wherein the presence of the Forssman antigen in the biological sample indicates a pathological condition in the human subject.

9. The *in-vitro* method of claim 8, wherein the pathological condition is **characterized by** an abnormal expression and/or presence of the Forssman antigen in the human subject.

10. The *in-vitro* method of any one of claims 8 or 9, wherein the pathological condition is a susceptibility of the subject to an infection with a pathologic microorganism.

11. The *in-vitro* method of any one of claims 8 or 9, wherein the pathological condition is cancer, such as a cancer disease **characterized by** the expression of the Forssman antigen in cells associated with the cancer disease, for example wherein the cancer disease is Forssman positive gastrointestinal cancer, a lung cancer or a breast cancer.

12. **A kit for detecting a target oligosaccharide antigen,** the kit comprising an antigen detection component comprising: i) a major capsid protein (VP1) of sheep polyomavirus (ShPyV), or an antigen binding fragment thereof, or (ii) a derivative of ShPyV-VP1 having an amino acid sequence which is at least 80% identical to the amino acid sequence of ShPyV-VP1 (SEQ ID NO:1), or an antigen binding fragment of the derivative of ShPyV-VP1.

13. **A use of a kit** according to claim 12 in a method of any one of claims 1 to 4.

14. **A use of an antigen detection component** in the detection of a Forssman antigen, wherein the antigen detection component comprises: i) a major capsid protein (VP1) of sheep polyomavirus (ShPyV), or an antigen binding fragment thereof, or (ii) a derivative of ShPyV-VP1 having an amino acid sequence which is at least 80% identical to the amino acid sequence of ShPyV-VP1 (SEQ ID NO:1), or an antigen binding fragment of the derivative of ShPyV-VP1.

15. **An antigen detection component** comprising: i) a major capsid protein (VP1) of sheep polyomavirus (ShPyV), or an antigen binding fragment thereof, or (ii) a derivative of ShPyV-VP1 having an amino acid sequence which is at least 80% identical to the amino acid sequence of ShPyV-VP1 (SEQ ID NO:1), or an antigen binding fragment of the derivative of ShPyV-VP1; wherein the antigen detection component comprises, or is coupled to, a detectable moiety.

## FIGURE 1

Legend:  ◯ - Gal    ▢ - GalNAc    ● - Glc    ■ - GlcNAc    ◆ - Neu

## FIGURE 2

Legend:  ◯ - Gal    ▢ - GalNAc    ● - Glc    ◆ - αNeu5Ac

FIGURE 3

A

Molecular interactions

B

Bias reduced omit electron desity → $F_P$ dilution → Dose response analysis

FIGURE 4

FIGURE 5

FIGURE 5 cont.

FIGURE 6

FIGURE 7

| Class | Structure | Name | Sequence | Gene |
|---|---|---|---|---|
| Globoseries GSL | ◯● R₁ | LacCer | Galβ→4Glcβ-Cer | B4GALT1 |
| | ◯◯● R₁ | Gb3Cer | Galα→4Galβ→4Glcβ-Cer | A4GALT |
| | ▢◯◯● R₁ | Gb4Cer (P antigen) | GalNAcβ→3Galα→4Galβ→4Glcβ-Cer | B3GALNT |
| | ▢▢◯◯● R₁ | Forssman antigen | GalNAcα→3GalNAcβ→3Galα→4Galβ→4Glcβ-Cer | GBGT1 |
| HBGA | ▲ ◯▨◯ R₁ | H antigen | Fucα→2Galβ→3/4GlcNAcβ→3Galβ-Cer | FUT1 |
| | ▲ ▢◯▨◯ R₁ | A antigen | GalNAcα→3(Fucα→2)Galβ→3/4GlcNAcβ→3Galβ-Cer | A3GALNT (ABO) |
| O-Glycans | ▢ R₂ | Tn antigen | GalNAcα-O-Ser/Thr | GALNT1 |
| | ◯▢ R₂ | T antigen | Galβ→3GalNAcα-O-Ser/Thr | C1GALT1 |

Legend:  ◯ - Gal   ● - Glc   ▢ - GalNAc   ▨ - GlcNAc   ▲ - Fuc

R₁ - Ceramide (Cer), R₂ - O-linked glycoprotein, GSL - Glycosphingolipids, HBGA - Histo blood group antigen, Gb3 - Globotriaose, Gb4 - Globotetraose

Europäisches
Patentamt

European
Patent Office

Office européen
des brevets

# EUROPEAN SEARCH REPORT

Application Number

EP 22 19 4458

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | STRÖH LUISA J. ET AL: "Structural Basis and Evolution of Glycan Receptor Specificities within the Polyomavirus Family", MBIO, vol. 11, no. 4, 25 August 2020 (2020-08-25), XP93023737, US ISSN: 2161-2129, DOI: 10.1128/mBio.00745-20 * page 12 - page 13 * * page 17 * * figure 6B * | 12,15 | INV. C07K14/025 G01N24/00 G01N33/50 G01N33/569 G01N33/574 G01N33/80 G01R33/44 G01R33/46 |
| A | JP 5 286467 B2 (UNIV SHIZUOKA NAT UNIV CORP; J OIL MILLS INC ET AL.) 11 September 2013 (2013-09-11) * paragraph [0007] * * claims 7-8 * | 1-15 | |
| A | Silva Tiago ET AL: "Expression of the Forssman antigen in gastrointestinal cancer", International Journal of Biomedical Laboratory Science (IJBLS), 1 January 2021 (2021-01-01), pages 21-27, XP093023792, Retrieved from the Internet: URL:https://www.ijbls.org/images/Research_ Article_-_Expression_of_the_Forssman_antig en_in_gastrointestinal_cancer.pdf [retrieved on 2023-02-14] * abstract * * Materials and Methods; page 22 - page 23 * | 1-15 | TECHNICAL FIELDS SEARCHED (IPC) C07K G01N G01R |

-/--

The present search report has been drawn up for all claims

| Place of search | Date of completion of the search | Examiner |
|---|---|---|
| The Hague | 21 February 2023 | Fabrowski, Piotr |

Europäisches
Patentamt

European
Patent Office

Office européen
des brevets

## EUROPEAN SEARCH REPORT

Application Number

EP 22 19 4458

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 0 399 464 A2 (ISHIKAWA EIJI [JP]; SUMITOMO PHARMA [JP]) 28 November 1990 (1990-11-28) * abstract * * page 3, line 30 – line 41 * | 1-15 | |
| A | US 2005/100958 A1 (STERN PETER L [GB] ET AL) 12 May 2005 (2005-05-12) * paragraph [0061] * | 1-15 | |
| A,D | SVENSSON LOLA ET AL: "Forssman expression on human erythrocytes: biochemical and genetic evidence of a new histo-blood group system", BLOOD, AMERICAN SOCIETY OF HEMATOLOGY, US, vol. 121, no. 8, 21 February 2013 (2013-02-21), pages 1459-1468, XP086510632, ISSN: 0006-4971, DOI: 10.1182/BLOOD-2012-10-455055 [retrieved on 2020-11-19] * Methods; page 1460 * | 1-15 | |

TECHNICAL FIELDS
SEARCHED     (IPC)

The present search report has been drawn up for all claims

| Place of search | Date of completion of the search | Examiner |
|---|---|---|
| The Hague | 21 February 2023 | Fabrowski, Piotr |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

...................................................................................................

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 22 19 4458

This annex lists the patent family members relating to the patent documents cited in the above-mentioned European search report.
The members are as contained in the European Patent Office EDP file on
The European Patent Office is in no way liable for these particulars which are merely given for the purpose of information.

21-02-2023

| Patent document cited in search report | | Publication date | Patent family member(s) | | Publication date |
|---|---|---|---|---|---|
| JP 5286467 | B2 | 11-09-2013 | JP | 5286467 B2 | 11-09-2013 |
| | | | JP | 2009209070 A | 17-09-2009 |
| EP 0399464 | A2 | 28-11-1990 | CA | 2017342 A1 | 24-11-1990 |
| | | | EP | 0399464 A2 | 28-11-1990 |
| | | | JP | H0373852 A | 28-03-1991 |
| US 2005100958 | A1 | 12-05-2005 | AU | 2003244823 A1 | 23-01-2004 |
| | | | CA | 2490163 A1 | 15-01-2004 |
| | | | EP | 1523680 A2 | 20-04-2005 |
| | | | US | 2005100958 A1 | 12-05-2005 |
| | | | WO | 2004005926 A2 | 15-01-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHENG, J. ; DECAPRIO, J. A. ; FLUCK, M. M. ; SCHAFFHAUSEN, B. S.** Cellular transformation by Simian Virus 40 and Murine Polyoma Virus T antigens. *Seminars in Cancer Biology,* 2009
- **FENG, H. ; SHUDA, M. ; CHANG, Y. ; MOORE, P. S.** Clonal integration of a polyomavirus in human Merkel cell carcinoma. *Science,* 2008, vol. 319, 1096-1100
- **CHANG, Y. ; MOORE, P. S.** Merkel cell carcinoma: A virus-induced human cancer. *Annual Review of Pathology: Mechanisms of Disease,* 2012
- **SALUNKE, D. M. ; CASPAR, D. L. D. ; GARCEA, R. L.** Self-assembly of purified polyomavirus capsid protein VP1. *Cell,* 1986
- **BAROUCH, D. H. ; HARRISON, S. C.** Interactions among the major and minor coat proteins of polyomavirus. *J. Virol.,* 1994, vol. 68, 3982-3989
- **CHEN, X. S. ; STEHLE, T. ; HARRISON, S. C.** Interaction of polyomavirus internal protein VP2 with the major capsid protein VP1 and implications for participation of VP2 in viral entry. *EMBO J.,* 1998, vol. 17, 3233-3240
- **TSAI, B. et al.** Gangliosides are receptors for murine polyoma virus and SV40. *EMBO J.,* 2003, vol. 22, 4346-4355
- **SMITH, A. E. ; LILIE, H. ; HELENIUS, A.** Ganglioside-dependent cell attachment and endocytosis of murine polyomavirus-like particles. *FEBS Lett.,* 2003
- **ERICKSON, K. D. ; GARCEA, R. L. ; TSAI, B.** Ganglioside GT1b Is a Putative Host Cell Receptor for the Merkel Cell Polyomavirus. *J. Virol.,* 2009, vol. 83, 10275-10279
- **GILBERT, J. et al.** Ganglioside GD1a Restores Infectibility to Mouse Cells Lacking Functional Receptors for Polyomavirus. *J. Virol.,* 2005, vol. 79, 615-618
- **BLAUM, B. S. ; STEHLE, T.** Sialic Acids in Nonenveloped Virus Infections. *Advances in Carbohydrate Chemistry and Biochemistry,* 2019, vol. 76, 65-111
- **NEU, U. et al.** Structures of merkel cell polyomavirus VP1 complexes define a sialic acid binding site required for infection. *PLoS Pathog.,* 2012, vol. 8, 8
- **NEU, U. et al.** A Structure-Guided Mutation in the Major Capsid Protein Retargets BK Polyomavirus. *PLoS Pathog.,* 2013, vol. 9, 01003688

- **NEU, U. et al.** Structures of B-Lymphotropic Polyomavirus VP1 in Complex with Oligosaccharide Ligands. *PLoS Pathog.,* 2013, vol. 9
- **STRÖH, L. J. et al.** Trichodysplasia spinulosa-Associated Polyomavirus Uses a Displaced Binding Site on VP1 to Engage Sialylated Glycolipids. *PLoS Pathog.,* 2015, vol. 11
- **STEHLE, T. ; HARRISON, S. C.** Crystal structures of murine polyomavirus in complex with straight-chain and branched-chain sialyloligosaccharide receptor fragments. *Structure,* 1996, vol. 4, 183-194
- **NEU, U. ; WOELLNER, K. ; GAUGLITZ, G. ; STEHLE, T.** Structural basis of GM1 ganglioside recognition by simian virus 40. *Proc. Natl. Acad. Sci. U. S. A.,* 2008, vol. 105, 5219-5224
- **KHAN, Z. M. et al.** Crystallographic and Glycan Microarray Analysis of Human Polyomavirus 9 VP1 Identifies N-Glycolyl Neuraminic Acid as a Receptor Candidate. *J. Virol.,* 2014, vol. 88, 6100-6111
- **STRÖH, L. J. et al.** Structural basis and evolution of glycan receptor specificities within the polyomavirus family. *MBio,* 2020, vol. 11, 1-21
- **O'HARA, S. D. ; STEHLE, T. ; GARCEA, R.** Glycan receptors of the Polyomaviridae: Structure, function, and pathogenesis. *Current Opinion in Virology,* 2014, vol. 7, 73-78
- **STEHLE, T. ; KHAN, Z. M.** Rules and Exceptions: Sialic Acid Variants and Their Role in Determining Viral Tropism. *J. Virol.,* 2014, vol. 88, 7696-7699
- **STEHLE, T. ; YAN, Y. ; BENJAMIN, T. L. ; HARRISON, S. C.** Structure of murine polyomavirus complexed with an oligosaccharide receptor fragment. *Nature,* 1994, vol. 369, 160-163
- **STEHLE, T. ; GAMBLIN, S. J. ; YAN, Y. ; HARRISON, S. C.** The structure of simian virus 40 refined at 3.1 Å resolution. *Structure,* 1996, vol. 4, 165-182
- **RUSTMEIER, N. H. ; STREBL, M. ; STEHLE, T.** The symmetry of viral sialic acid binding sites-implications for antiviral strategies. *Viruses,* 2019, vol. 11, 947
- **ELPHICK, G. F. et al.** The human polyomavirus, JCV, uses serotonin receptors to infect cells. *Science,* 2004, vol. 306, 1380-1383
- **ASSETTA, B. et al.** 5-HT2 Receptors Facilitate JC Polyomavirus Entry. *J. Virol.,* 2013, vol. 87, 13490

- **SCHOWALTER, R. M. ; PASTRANA, D. V. ; BUCK, C. B.** Glycosaminoglycans and sialylated glycans sequentially facilitate merkel cell polyomavirus infectious entry. *PLoS Pathog.,* 2011, vol. 7, 1002161
- **HURDISS, D. L. ; FRANK, M. ; SNOWDEN, J. S. ; MACDONALD, A. ; RANSON, N. A.** The Structure of an Infectious Human Polyomavirus and Its Interactions with Cellular Receptors. *Structure,* 2018, vol. 26, 839
- **GEOGHEGAN, E. M. et al.** Infectious Entry and Neutralization of Pathogenic JC Polyomaviruses. *Cell Rep.,* 2017, vol. 21, 1169-1179
- **ASSETTA, B. et al.** Genetic and Functional Dissection of the Role of Individual 5-HT2 Receptors as Entry Receptors for JC Polyomavirus. *Cell Rep.,* 2019, vol. 27, 1960-1966.06
- **BUCK, C. B. et al.** The Ancient Evolutionary History of Polyomaviruses. *PLoS Pathog.,* 2016, vol. 12, 1005574
- **CALVIGNAC-SPENCER, S. et al.** A taxonomy update for the family Polyomaviridae. *Arch. Virol.,* 2016, vol. 161, 1739-1750
- **TORRES, C.** Evolution and molecular epidemiology of polyomaviruses. *Infection, Genetics and Evolution,* 2020, vol. 79, 104150
- **VARKI, A. et al.** Essentials of Glycobiology. *Cold Spring Harb.,* 2017, vol. 823
- **SONNENBERG, A. et al.** Monoclonal antibodies detecting different epitopes on the Forssman glycolipid hapten. *J. Immunol.,* 1986
- **HASLAM, D. B. ; BAENZIGER, J. U. ; BAENZIGERT, J. U. ; BAENZIGER, J. U.** Expression cloning of Forssman glycolipid synthetase: A novel member of the histo-blood group ABO gene family. *Proc. Natl. Acad. Sci. U. S. A.,* 1996, vol. 93, 10697-10702
- **YAMAMOTO, F. et al.** An integrative evolution theory of histo-blood group ABO and related genes. *Sci. Rep.,* 2014, vol. 4, 6601
- **YAMAMOTO, M. ; CID, E. ; YAMAMOTO, F.** Molecular genetic basis of the human Forssman glycolipid antigen negativity. *Sci. Rep.,* 2012, vol. 2, 975
- **TANAKA, N. ; LEDUC, E. H. A.** Study of the Cellular Distribution of Forssman Antigen in Various Species. *J. Immunol.,* 1956, vol. 77, 198-212
- **STROMBERG, N. et al.** Host-specificity of uropathogenic Escherichia coli depends on differences in binding specificity to Gal$\alpha$1-4Gal-containing isoreceptors. *EMBO J.,* 1990, vol. 9, 2001-2010
- **STROMBERG, N. ; NYHOLM, P. G. ; PASCHER, I. ; NORMARK, S.** Saccharide orientation at the cell surface affects glycolipid receptor function. *Proc. Natl. Acad. Sci. U. S. A.,* 1991, vol. 88, 9340-9344
- **JOHANSON, I. ; LINDSTEDT, R. ; SVANBORG, C.** Roles of the pap- and prs-encoded adhesins in Escherichia coli adherence to human uroepithelial cells. *Infection and Immunity,* 1992, vol. 60, 3416-3422
- **LANNE, B. et al.** Glycoconjugate Receptors for P-fimbriated Escherichia coli in the Mouse: AN ANIMAL MODEL OF URINARY TRACT INFECTION *. *J. Biol. Chem.,* 1995, vol. 270, 9017-9025
- **MÜTHING, J. et al.** Promiscuous Shiga toxin 2e and its intimate relationship to Forssman. *Glycobiology,* 2012, vol. 22, 849-862
- **ELLIOTT, S. P. ; YU, M. ; XU, H. ; HASLAM, D. B.** Forssman Synthetase Expression Results in Diminished Shiga Toxin Susceptibility: A Role for Glycolipids in Determining Host-Microbe Interactions. *Infect. Immun.,* 2003, vol. 71, 6543-6552
- **YOUNG, W. W. ; HAKOMORI, S.-I. ; LEVINE, P.** Characterization of Anti-Forssman (ANTI-Fs). *Antibodies in Human Sera: Their Specificity and Possible Changes in Patients with Cancer,* 1979, vol. 123
- **SVENSSON, L. et al.** Forssman expression on human erythrocytes: Biochemical and genetic evidence of a new histo-blood group system. *Blood,* 2013, vol. 121, 1459-1468
- The Appearance of Forssman Hapten in Human Tumor. **KAWANAMI, J.** Journal of Biochemistry. Oxford University Press, 1972, vol. 72, 783-785
- **HAKOMORI, S. ; WANG, S. M. ; YOUNG, W. W.** Isoantigenic expression of Forssman glycolipid in human gastric and colonic mucosa: Its possible identity with 'A like antigen' in human cancer. *Proc. Natl. Acad. Sci. U. S. A.,* 1977, vol. 74, 3023-3027
- **YODA, Y. ; ISHIBASHI, T. ; MAKITA, A.** Isolation, Characterization, and Biosynthesis of Forssman Antigen in Human Lung and Lung Carcinoma. *J. Biochem.,* 1980, vol. 88, 1887-1894
- **TANIGUCHI, N. ; YOKOSAWA, N. ; NARITA, M. ; MITSUYAMA, T. ; MAKITA, A.** Expression of forssman antigen synthesis and degradation in human lung cancer. *J. Natl. Cancer Inst.,* 1981, vol. 67, 577-583
- **MORI, T. et al.** Forssman antibody levels in sera of cancer patients. *Immunol. Invest.,* 1982, vol. 11, 217-225
- **MORI, T. ; SUDO, T. ; KANO, K.** Expression of Heterophil Forssman Antigen on Cultured Malignant Cell Lines. *JNCI J. Natl. Cancer Inst.,* 1983, vol. 70, 811-818
- **HAKOMORI, S.** Tumor-Associated Carbohydrate Antigens. *Annual review of immunology,* 1984, vol. 2, 103-126
- **UEMURA, K. ; HATTORI, H. ; ONO, K. ; OGATA, H. ; TAKETOMI, T.** Expression of Forssman glycolipid and blood group-related antigens A, Le(x) and Le(y) in human gastric cancer and in fetal tissues. *Jpn. J. Exp. Med.,* 1989, vol. 59, 239-249

- **ONO, K. et al.** Expression of Forssman antigen in human large intestine. *J. Histochem. Cytochem.,* 1994, vol. 42, 659-665
- **CID, E. ; YAMAMOTO, M. ; YAMAMOTO, F.** Mixed-Up Sugars: Glycosyltransferase Cross-Reactivity in Cancerous Tissues and Their Therapeutic Targeting. *ChemBioChem,* 2021
- **HIRAYAMA, R. et al.** Changes in Serum Levels of Forssman-Like Antibody in Patients With Gastric Cancer. *Cancer,* 1989, vol. 63, 1528-1528
- **MAYER, M. ; MEYER, B.** Group epitope mapping by saturation transfer difference NMR to identify segments of a ligand in direct contact with a protein receptor. *J. Am. Chem. Soc.,* 2001
- **BLAUM, B. S. ; NEU, U. ; PETERS, T. ; STEHLE, T.** Spin ballet for sweet encounters: Saturation-transfer difference NMR and X-ray crystallography complement each other in the elucidation of protein-glycan interactions. *Acta Crystallographica Section F: Structural Biology Communications,* 2018, vol. 74, 451-462
- **GRÖNBERG, G. ; NILSSON, U. ; BOCK, K. ; MAGNUSSON, G.** Nuclear magnetic resonance and conformational investigations of the pentasaccharide of the Forssman antigen and overlapping di-, tri-, and tetra-saccharide sequences. *Carbohydr. Res.,* 1994, vol. 257, 35-54
- **NAGAE, M. et al.** Structural Analysis of the Human Galectin-9 N-terminal Carbohydrate Recognition Domain Reveals Unexpected Properties that Differ from the Mouse Orthologue. *J. Mol. Biol.,* 2008, vol. 375, 119-135
- **HAMELRYCK, T. W. et al.** Carbohydrate binding, quaternary structure and a novel hydrophobic binding site in two legume lectin oligomers from Dolichos biflorus. *J. Mol. Biol.,* 1999, vol. 286, 1161-1177
- **LESCAR, J. et al.** Structural basis for recognition of breast and colon cancer epitopes Tn antigen and Forssman disaccharide by Helix pomatia lectin. *Glycobiology,* 2007, vol. 17, 1077-1083
- **KITA, A. et al.** Crystal structure of octocoral lectin SLL-2 complexed with Forssman antigen tetrasaccharide. *Glycobiology,* 2017, vol. 27, 696-700
- **WU, A. M. ; SUGII, S.** Coding and classification of d-galactose, N-acetyl-d-galactosamine, and β-d-Galp-[1→(4)]-β-d-GlcpNAc, specificities of applied lectins. *Carbohydr. Res.,* 1991, vol. 213, 127-143
- **RAMBARUTH, N. D. ; GREENWELL, P. ; DWEK, M. V.** The lectin Helix pomatia agglutinin recognizes O-GlcNAc containing glycoproteins in human breast cancer. *Glycobiology,* 2012, vol. 22, 839-848
- **PARAMESWARAN, R. et al.** Binding of aberrant glycoproteins recognizable by Helix pomatia agglutinin in adrenal cancers. *BJS Open,* 2018, vol. 2, 353-359
- **JIMBO, M. et al.** Possible involvement of glycolipids in lectin-mediated cellular transformation of symbiotic microalgae in corals. *J. Exp. Mar. Bio. Ecol.,* 2013, vol. 439, 129-135
- **HAMMARSTROM, S. ; KABAT, E. A. ; HAMMARSTROMF, S. ; KABAT, E. A.** Purification and Characterization of a Blood-Group A Reactive Hemagglutinin from the Snail Helix pomatia and a Study of Its Combining Site*. *Biochemistry,* 1969, vol. 8, 2696-2705
- **TORRES, B. V. ; SMITH, D. F.** Purification of Forssman and human blood group A glycolipids by affinity chromatography on immobilized Helix pomatia lectin. *Anal. Biochem.,* 1988, vol. 170, 209-219
- **SCHUMACHER, U. et al.** Helix Pomatia Agglutinin Binding Is a Useful Prognostic Indicator in Colorectal Carcinoma. *Cancer,* 1994, vol. 74, 3104-3107
- **SCHUMACHER, U. ; ADAM, E. ; BROOKS, S. A. ; LEATHERN, A. J.** Lectin-binding properties of human breast cancer cell lines and human milk with particular reference to Helix pomatia agglutinin. *J. Histochem. Cytochem.,* 1995, vol. 43, 275-281
- **SCHUMACHER, U.** The use of the lectin Helix pomatia agglutinin (HPA) as a prognostic indicator and as a tool in cancer research. *Histol. Histopathol.,* 1999, vol. 14, 217-226
- **DWEK, M. V. et al.** Helix pomatia agglutinin lectin-binding oligosaccharides of aggressive breast cancer. *Int. J. Cancer (Pred. Oncol,* 2001, vol. 95, 79-85
- **SANCHEZ, J. F. et al.** Biochemical and Structural Analysis of Helix pomatia Agglutinin: A HEXAMERIC LECTIN WITH A NOVEL FOLD. *J. Biol. Chem.,* 2006, vol. 281, 20171-20180
- **EHLERS, B. et al.** Novel Polyomaviruses in Mammals from Multiple Orders and Reassessment of Polyomavirus Evolution and Taxonomy. *Viruses,* 2019, vol. 11, 930
- **NEU, U. ; BAUER, J. ; STEHLE, T.** Viruses and sialic acids: Rules of engagement. *Current Opinion in Structural Biology,* 2011, vol. 21, 610-618
- **SAUTER, N. K. et al.** Hemagglutinins from Two Influenza Virus Variants Bind to Sialic Acid Derivatives with Millimolar Dissociation Constants: A 500-MHz Proton Nuclear Magnetic Resonance Study. *Biochemistry,* 1989, vol. 28, 8388-8396
- **BURGER, M. M.** Forssman Antigen exposed on Surface Membrane after Viral Transformation. *Nat. New Biol.,* 1971, vol. 23121 (231), 125-126
- **KARJALAINEN, H. E. ; MÄNTYJÄRVI, R. A.** FORSSMAN ANTIGEN IN BK VIRUS-INDUCED TUMOR CELL LINES. *Acta Pathol. Microbiol. Scand. Sect. C Immunol.,* 1981, vol. 89C, 49-54
- **JU, T. ; OTTO, V. I. ; CUMMINGS, R. D.** The Tn Antigen-Structural Simplicity and Biological Complexity. *Angew. Chemie Int. Ed.,* 2011, vol. 50, 1770-1791

- **UHLENBRUCK, G. ; PROKOP, O.** An Agglutinin from Helix Pomatia,which Reacts with Terminal N-Acetyl-D-Galactosamine. *Vox Sang.,* 1966, vol. 11, 519-520
- **STELOS, P. ; GRAVES TALIAFERRO, L. ; D, P. A.** Comparative Study of Rabbit Hemolysins to Various Antigens: III. Chromatographic Analysis of Forssman Hemolysins Induced by Various Antigens. *Source J. Infect. Dis.,* 1961, vol. 108, 113-119
- **KABSCH, W. et al.** *XDS. Acta Crystallogr. Sect. D Biol. Crystallogr.,* 2010
- **MCCOY, A. J. et al.** Phaser crystallographic software. *J. Appl. Crystallogr.,* 2007, vol. 40, 658-674
- **LIEBSCHNER, D. et al.** Macromolecular structure determination using X-rays, neutrons and electrons: Recent developments in Phenix. *Acta Crystallogr. Sect. D Struct. Biol.,* 2019

- **MURSHUDOV, G. N. et al.** REFMAC5 for the refinement of macromolecular crystal structures. *Acta Crystallogr. Sect. D Biol. Crystallogr.,* 2011
- **EMSLEY, P. ; COWTAN, K.** Coot: Model-building tools for molecular graphics. *Acta Crystallogr. Sect. D Biol. Crystallogr.,* 2004
- **BUCH, M. H. C. C. et al.** Structural and Functional Analysis of Murine Polyomavirus Capsid Proteins Establish the Determinants of Ligand Recognition and Pathogenicity. *PLoS Pathog.,* 2015, vol. 11, 01005104
- **HOWELL, P. L.** Identification of heavy-atom derivatives by normal probability methods. *J. Appl. Crystallogr.,* 1992, vol. 25, 81-86
- **KLEYWEGT, G. J. ; JONES, T. A.** xdlMAPMAN and xdlDATAMAN - Programs for reformatting, analysis and manipulation of biomacromolecular electron-density maps and reflection data sets. *Acta Crystallogr. Sect. D Biol. Crystallogr.,* 1996, vol. 52, 826-828